# EUROPEAN PATENT APPLICATION

(11) **EP 4 438 623 A1**
(43) Date of publication of application: **02.10.2024**
(21) Application number: 23164787.6
(22) Date of filing: 28.03.2023
(51) Int. Cl.: C07K 16/10, A61K 39/395, A61P 31/14, C12N 15/13

(54) **ENGINEERED ANTI-SPIKE IGG3 ANTIBODIES**

(71) Applicant: Tanea Medical AB, 753 20 Uppsala (SE)
(72) Inventor: NORDENFELT, Bill Pontus, Uppsala (SE); BAHNAN, Wael Gabriel, 75320 Uppsala (SE); IZADI, Arman, 75320 Uppsala (SE)
(74) Representative: Zacco Denmark A/S

(57) **Abstract**

The present invention generally relates to immunology and antibody engineering, and specifically to engineered antibodies which have improved properties. More specifically, the present invention provides an engineered antibody comprising an lgG3 subclass specific domain and a variable domain having a viral spike protein binding part.

## Description

### Technical field of the invention

The present invention relates to the fields of immunology, vaccines and pharmaceuticals products. Specifically the invention relates to antibodies, pharmaceutical compositions, antibody engineering, viral vaccines and methods for preparing and using same.

### Background of the invention

SARS-CoV-2 has caused millions of deaths worldwide since it first emerged in 2019 ¹. Early in the pandemic monoclonal antibodies (mAbs) were successfully used as therapeutics ². These antibodies disrupted the ACE2 receptor-spike receptor-binding domain (RBD) interaction, thus neutralizing the virus and protecting the host ³. Neutralizing antibodies against the SARS-CoV-2 spike protein constitute only a fraction of the antibody immune response ⁴. It has previously been shown that a non-neutralizing but opsonic mAb was as good as a potent neutralizing mAb in protecting SARS-CoV-2 infected animals ⁵. These findings are in line with other studies showing that Fc-effector functions are crucial for viral control ^{6,7}. Indeed, the protection granted by vaccines against mutated variants like Omicron can partially be explained by intact antibody Fc-effector functions directed against non-RBD sites or RBD sites not subjected to mutations ⁸.

Antibody class and subclass play a significant role in determining functional outcomes, especially in the context of Fc and complement receptor-mediated phagocytosis. The human IgG class has four subclasses defined by differences in the constant domains of the heavy chain ⁹. The constant domain has historically been considered independent of the variable domain and is responsible only for antibody effector functions. However, growing evidence with subclass-switched antibodies shows that the constant domain seems to influence the affinity to the target antigen ¹⁰. Nonetheless, the antibody subclass constant domain research has mainly focused on the affinity to Fc-receptors and complement activation. IgG1 and IgG3 are the most potent activators of Fc gamma receptors on immune cells, likely due to their high affinity to these receptors ¹¹. IgG3 is also the most potent activator of the classical complement pathway, followed by IgG1, 2, and 4 ^{12,13}. In the case of HIV-1, IgG3 has a stronger opsonic ability compared to IgG1 and mediates stronger Fc-mediated phagocytosis of beads conjugated with the gp140 antigen; this is despite IgG3 and IgG1 anti-HIV antibodies having similar affinities to their targets ¹⁴. The difference in function is attributed to the more extended hinge region on IgG3, which grants its Fc-tail more spatial flexibility. In a related study, exchanging the IgG1 hinge with an IgG3 hinge enhanced intracellular immunity against adenovirus infection, supporting the flexibility hypothesis¹⁵. The IgG subclass, therefore, is a determining factor in optimizing the immune function of monoclonal antibodies against viral pathogens. Recently it was shown that subclass-switching to IgG3 can enhance the neutralization of SARS-CoV-2¹⁶ and that IgG3 backbones can promote ACE2-Fc protein effector function¹⁷. Several aspects are still unknown, such as the effect of the IgG subclass on antibody affinity to the spike protein, its impact on complement activation, its relationship to subsequent Fc-mediated immune functions, and how combining various anti-Spike antibodies may influence Fc-mediated antibody functions.

The basis for mAb therapy against SARS-CoV-2 rests in the ability of neutralizing mAbs to bind to the RBD, thus disrupting the spike protein's ability to interact with the ACE2 receptor. However, due to the rise of extensive mutations found in the RBD of the emerging variants of concern (VOC), most of these antibodies lost binding leading to loss of function and treatment failure^{20,21}. This has led to alternative strategies by utilizing combinations of neutralizing antibodies (nAbs) to treat these new variants²² or find universally neutralizing antibodies²³.

It was previously shown that an ACE2-Fc construct utilizing an IgG3 backbone showed higher potency than its IgG1 counterpart *in vitro* ¹⁷. In non-SARS-CoV-2 contexts, IgG3 mAbs have been shown to be efficient at mediating Fc-mediated phagocytosis of HIV virions ¹⁴. A similar benefit has been observed with enhanced intracellular activity against an adenovirus via the TRIM21-receptor¹⁵.

WO2020/254591 discloses immunoreceptors comprising one or more IgGS middle hinge repeat domain motifs, wherein the immunoreceptor does not comprise an IgGS CH2 and/or CH3 domain.

There exist some biological limitations to IgG3's utility as a drug *in vivo.* Of the four subclasses, IgG3 has the shortest half-life ⁹ due to lower affinity to the neonatal Fc-receptor ⁹. Through Fc-engineering, the affinity to the Fc-neonatal receptor can be increased without diminishment to Fc-effector functions ^{30,31}. This alteration has been shown to be crucial for *in vivo* protection in a mouse model ³⁰.

There is a need for improved monoclonal antibodies for the treatment and prevention of viral infections, such as infections from SARS-CoV-2. In particular there is a need for monoclonal antibodies and composition containing such antibodies which are more efficient and which protects against more viral variants.

### Summary of the invention

The object of the present invention is to overcome certain drawbacks in antibodies and their use in the treatment or prevention of viral infections. This is achieved by the present inventors who have designed novel antibodies (monoclonal antibodies, mAbs) which are engineered forms of antibodies prepared by immunization using a viral spike protein as antigen.

The present inventors unexpectedly and surprisingly found that subclass switching an IgG1 antibody into an IgG3 antibody enhances both Fc- and complement receptor (CR)-mediated phagocytosis.

The present inventors furhter surprisingly found that engineering an IgG1 antibody by substituting the IgG1 hinge with the IgG3 hinge facilitated novel antibodies having improved properties. Hence, altering the constant domain of an antibody can strongly potentiate the immune response against e.g. the SARS-Cov-2 virus.

It has furthermore surprisingly been found that antibody immune function is enhanced when combining antibodies in cocktails containing several different antibodies. Oligoclonal cocktails of IgG3 subclass mAbs are found to be more potent than even the best-performing monoclonal antibodies in both Fc- and CR-mediated phagocytosis.

Hence, antibody subclass engineering and cocktail formation modulates the efficacy of antibodies as therapeutics against viral infections.

Based on the foregoing findings, the present invention provides in a first aspect an engineered antibody comprising an lgG3 subclass specific domain and a variable domain having a viral spike protein binding part.

The present invention further provides a composition comprising at least two different engineered antibodies according to the invention.

The present invention further provides the use of the engineered antibody according to the invention for preventing, treating or alleviating a viral infection caused by a virus comprising said spike protein.

The present invention further provides the use of the engineered antibody according to the invention for Fc-mediated phagocytosis of a virus comprising said spike protein.

The present invention further provides the use of the composition according to the invention for activating the complement pathway.

The present invention further provides the use of the composition according to the invention for Fc-mediated phagocytosis of a virus comprising said spike protein.

The present invention further provides a method for preparing an engineered antibody according to the invention, comprising the steps:
- immunization of a host organism with said viral spike protein or a protein comprising said viral spike protein,
- isolation of at least one viral spike protein reactive B-cell to isolate an IgG1 antibody binding to said viral spike protein,
- preparing at least one recombinant DNA molecule which encodes a modified version of said IgG1 antibody comprising the replacing of the IgG1 subclass specific domain of said isolated IgG1 antibody with the IgG3 subclass specific domain,
- expressing said at least one recombinant DNA molecule in a suitable host cell,
to obtain said engineered antibody.

### Brief description of the figures

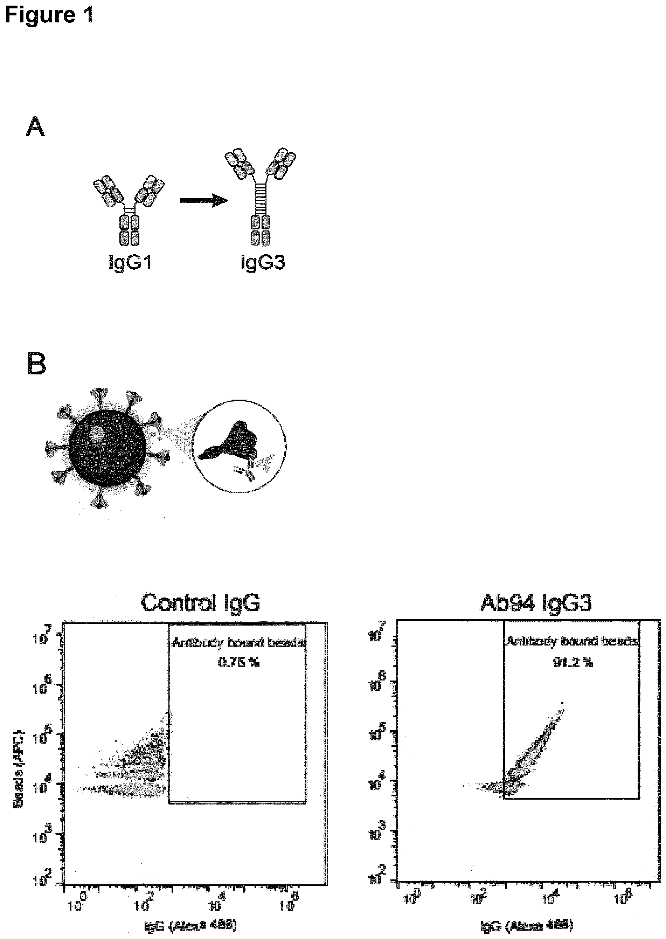

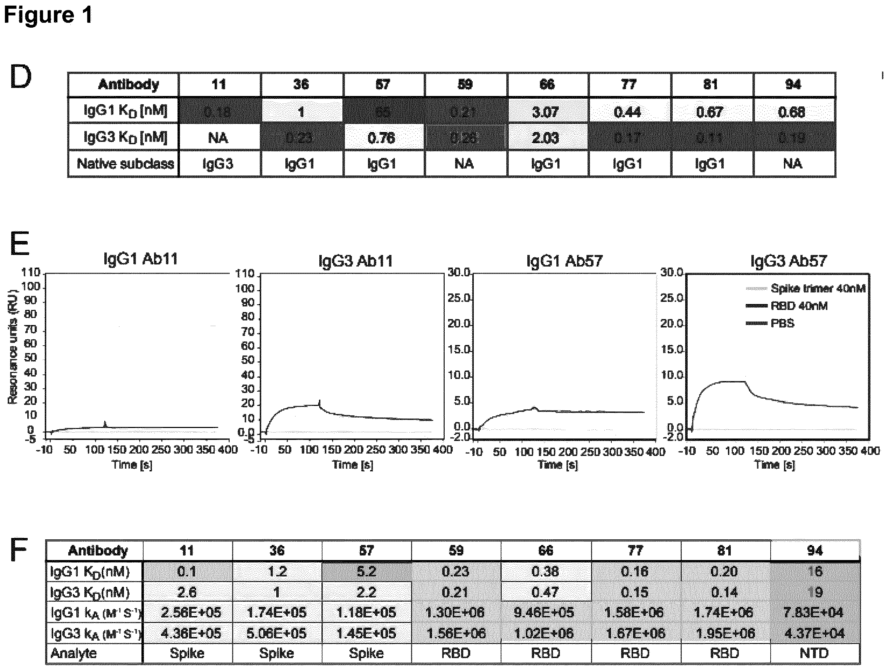

**Figure 1A-F****. Switching the IgG1 constant domain to IgG3 can alter the avidity for Spike protein. A** Schematic of the heavy and light chain plasmids containing the variable and constant domains. The generation of IgG3 mAbs entails switching the constant domain of the heavy chain from IgG1 (blue) to IgG3 (orange). **B** Spike-coated microspheres are used as a model for SARS-CoV-2 virions. Antibody binding assay is done by opsonizing Spike beads with mAbs and adding a secondary Alexa 488-conjugated antibody that reports IgG binding to Spike beads. **C** Binding curves showing the percentage of IgG positive Spike beads as a function of IgG concentration. Each clone is shown with both subclasses present (IgG1 and IgG3). Three independent experiments were performed with the mean value shown in the graph and error bars representing the SEM. **D** Table summarizing the subclass K_{D}-values and original subclass for each clone. Avidity was calculated by using a non-linear regression model in GraphPad Prism. **E.** Surface plasmon resonance-based binding kinetics with whole spike protein (trimer). Binding of 40 nM Spike-protein (thinner line) to immobilized IgG compared to 40nM RBD (dense line) and PBS (lower curve) for clones 11, 36, 57 and their respective subclasses. **F** Table with K_{D}-values and K_{A} for the different subclasses across all clones (11-94) and what analyte (spike, RBD or NTD) was used to measure it with the SPR-based assay. Statistical analysis was performed using one-way ANOVA with multiple comparisons and Tukey for correction. P-value above 0.05 denotes ns, p-value below 0.05 denotes ^{∗}, p-value below 0.01 denotes ^{∗∗}, p-value below 0.001 denotes ^{∗∗∗} and p-value below 0.0001 denotes ^{∗∗∗∗}. **G** ELISA data with spike-coated wells and bound IgG is shown for clones 11 and 57 and their respective subclasses. Three independent experiments were performed with titration curves to plot binding curves. Here one representative experiment with 4 technical repeats is shown with mean value and error bars representing the SEM.

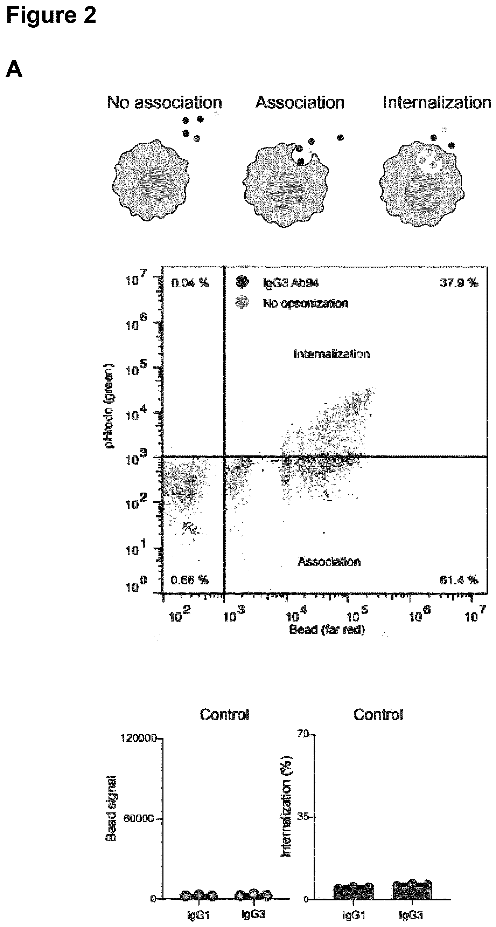

**Figure 2A-E****. IgG3 antibodies are superior to IgG1 antibodies at inducing Fc-mediated phagocytosis further potentiated when used as oligoclonal cocktails. A** Illustration showing the three different phagocytic states cells can be in and how they are seen in flow cytometric gates: first depicting non-associated cells, second associated but not internalized, and third with internalized beads. The gate to the right shows a double positive population signifying internalized beads. Furthest to the right is a graph showing IgG isotype controls. Y-axis shows the percentage of THP-1 cells with internalized beads. The same experimental conditions were used as in **B-C. B** IgG1 and IgG3 variants of each antibody clone mediate phagocytosis to varying degrees. The bar graph shows the percentage of cells with internalized beads. 10 µg/ml of the corresponding mAb was used to opsonize the beads (3 million beads per 100000 phagocytes, a multiplicity of prey 30 (MOP 30). **C** Comparison between IgG1 and IgG3 for each clone with bead signal average for the whole THP-1 population. **D** The graph depicts how monoclonals compare to the cocktails in terms of bead quantity that is being phagocytosed by the whole cell population. The table to the right shows the mean values for the median bead signal (fluorescence intensity) for each treatment. **E** Comparison between IgG1 and IgG3 monoclonal antibodies and cocktails in terms of percentage of THP-1 cells with internalized beads. The table to the right summarizes the data from the graph with mean values from three experiments. In **D-E** 1.5 million beads per 100.000 phagocytes (MOP 15) were opsonized with 5 µg/ml of either monoclonal antibody or IgG cocktail. Statistical analysis was performed with a two-tailed Welch's t-test in **B-C**. For **D-E**, statistical analysis was done with one-way ANOVA with multiple comparisons corrected with Tukey's test. P-value below 0.05 is denoted by ^{∗}, p-value below 0.01 is denoted by ^{∗∗}, and above 0.05 is denoted as ns. Three independent experiments were performed. Bars represent mean values, and error bars represent SEM. Three independent experiments were done for all experiments.

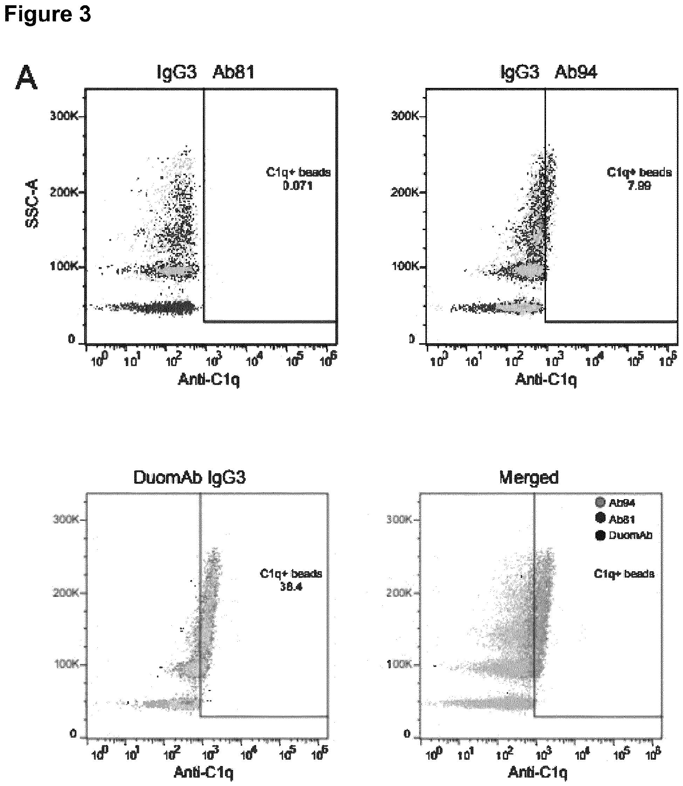

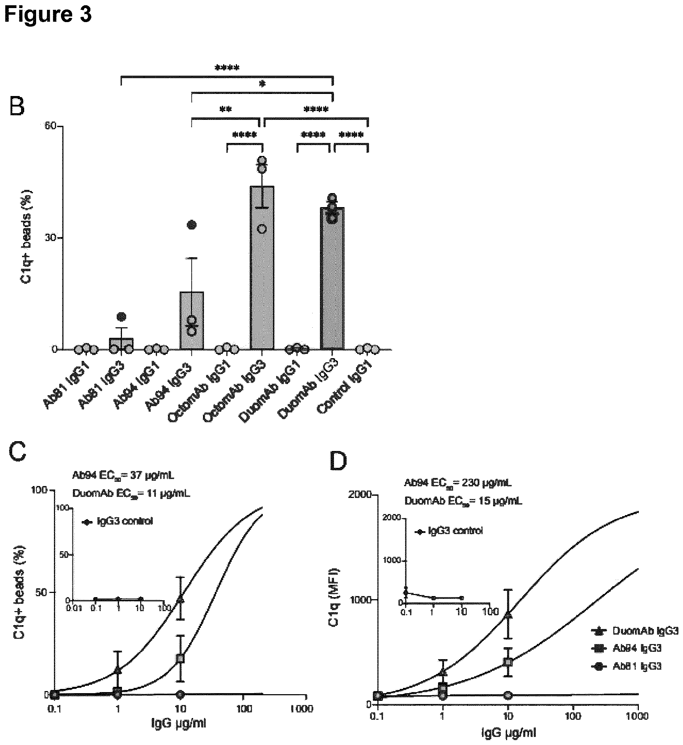

**Figure 3A-F****. IgG subclass and oligoclonality affect complement activation and phagocytosis of spike beads by neutrophils. A** Individual gates for C1q deposition for Ab81, Ab94, and OctomAb IgG3 at 10 µg/mL. The far-right panel shows all three plots merged. **B** Shows data on C1q deposition for the indicated monoclonals and oligoclonal cocktails. **C** Percentage of C1q deposition as a function of antibody concentration with EC₅₀ values in the graph. **D** Fluorescent signal of deposited C1q as a function of antibody concentration with EC₅₀ values present in the graph. In **C-D,** IgG3 isotype control data is shown as a small graph in each respective graph. **E** Neutrophils with internalized spike-beads comparing different treatments and heat-inactivated serum (HI) with complement-active serum (C+). **F** Bead signal of neutrophils associated with beads. **E-F** Isotype controls are shown furthest to the right. Three independent experiments were performed for **A-E.** For **A-E** Statistical analysis was performed with one-way ANOVA and correction for multiple comparisons was done with Tukey's test. ^{∗} denotes p-value below 0.05, ^{∗∗} denotes p-value below 0.01, ^{∗∗∗} denotes p-value below 0.001 and ns denotes a p-value above 0.05.

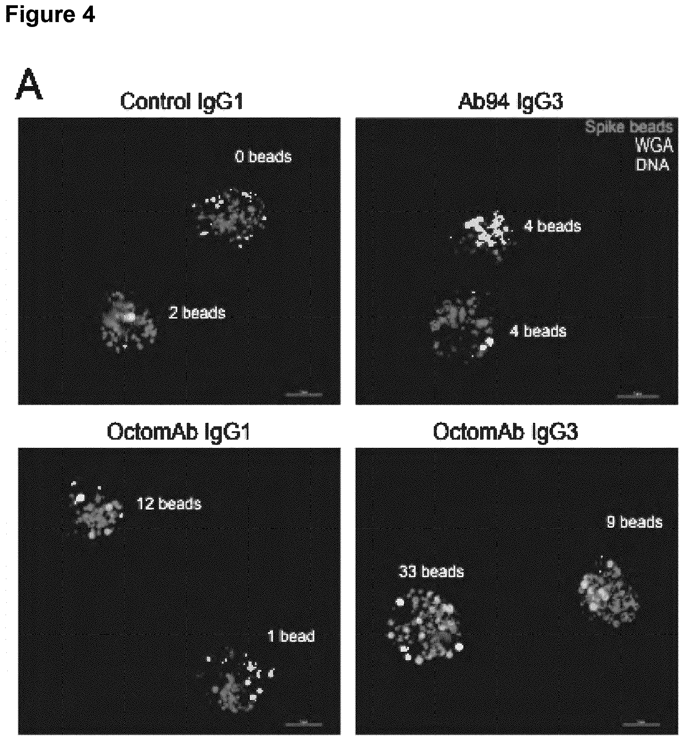

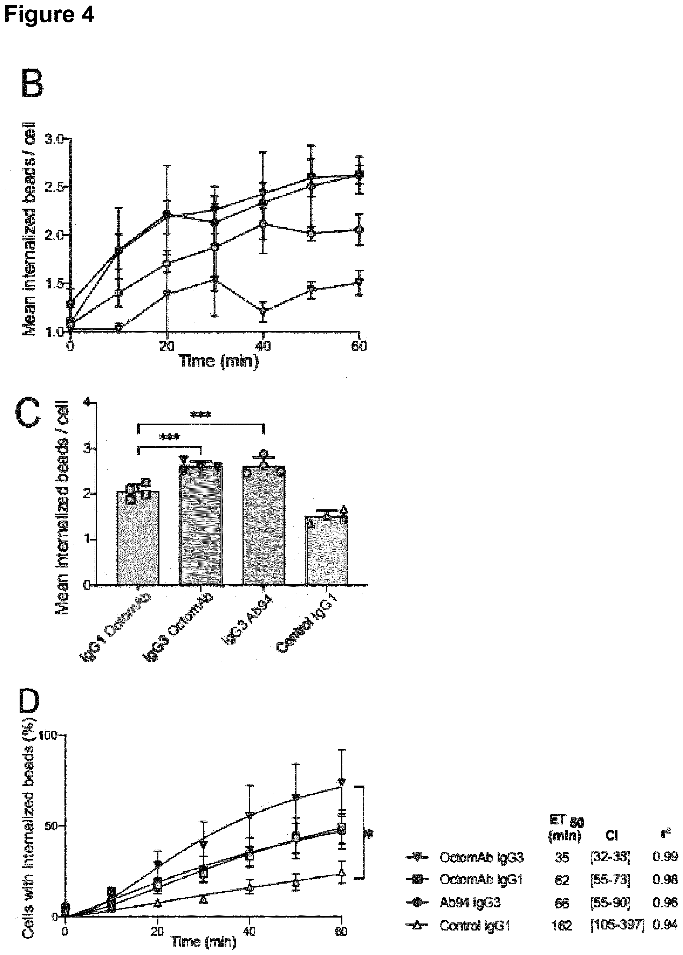

**Figure 4A-J****. Fc-mediated protection in vivo with hACE2-K18 mice and live imaging of human neutrophil phagocytosis. A** Super-resolution structured illumination microscopy images showing human neutrophil phagocytosis of Spike beads opsonized with indicated treatments. Beads are seen in the picture (stain), cell surface (Alexa 594-WGA), and DNA (Hoechst). pHrodo green was used as an internalization marker not seen in the image. Different antibody treatments result in differences in the number of beads internalized and the percentage of cells internalizing beads. SIM images are seen in a maximum-intensity projection of Z-stacks. Example images are shown from one experiment out of four. Scale bars in the lower right corners are 5 µm. **B** Change in the average number of beads for the neutrophils with internalized beads over time. The bar graph in **C** shows the results after 60 minutes. **D** Temporal change in phagocytosis is monitored and displayed in terms of neutrophils with internalized Spike beads. Below the graphs, ET₅₀ values are presented in the table with 95 % confidence intervals in brackets. **E** Humanized ACE2 mice were prophylactically injected intraperitoneally with antibody treatment or vehicle. Eight hours post-treatment, mice were infected intranasally with SARS-CoV-2 (Wuhan strain). Body weights were measured over ten days. **F** Mean relative body weight for each group from day 0-7, the red dashed line highlights when all mice in the control group were deceased. **G** Status of each mouse (alive/euthanized) in each treatment group at day 6. **H** Relative body weight for each mouse in each treatment group at day 6, with euthanized mice set to 0. **I** Survival curves for each treatment group over the 10-day experiment. **J** Terminal titers of viral load were analyzed from BAL-fluid from euthanized mice, irrespective of which day of death. qPCR was done on BAL-fluid, and the cycle threshold was recorded and graphed. The median value is shown. The mean value is shown for figures B-D and F-J, with error bars being SEM. Statistical analysis was performed by one-way ANOVA, and correction for multiple comparisons was done with Tukey's test. ^{∗} denotes p-value below 0.05, ^{∗∗} denotes below 0.01, ^{∗∗∗} denotes below 0.001, ^{∗∗∗∗} denotes below 0.0001, and ns denotes p-values above 0.05 and are not shown. In **B-D,** the data is from four independent experiments with four different donors.

### Detailed description of the invention

Unless specifically defined herein, all technical and scientific terms used have the same meaning as commonly understood by a skilled artisan in the fields of immunology, biochemistry, genetics, and microbiology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, and recombinant DNA, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory). Kuby Immunology (2018, J Punt & S Stranford). Therapeutic Antibody Engineering (2012, 1st Ed), W Strohl & L Strohl.

The present invention provides an engineered antibody comprising an IgG3 hinge and a variable domain having a viral spike protein binding part.

The present invention provides and is further characterized by the following items:
1. An engineered antibody comprising an IgG3 hinge and a variable domain having a viral spike protein binding part.
2. The engineered antibody according to item 1, wherein the Fc region is an IgG3 region.
3. The engineered antibody according to item 2, wherein the Fc region comprises the IgG3 heavy chain constant domain SEQ ID NO:1 or a variant thereof having at least 90% identity, at least 95% identity, at least 98% identity or 100% identity to SEQ ID NO:1.
4. The engineered antibody according to any one of items 1-2, which comprises an IgG3 Fc region comprising one or more substitutions selected from the group consisting of R435H, N392K and M397V.
5. The engineered antibody according to item 4, wherein the IgG3 region comprises the three substitutions R435H, N392K and M397V.
6. The engineered antibody according to any of items 3-5, wherein said IgG3 region comprises a polypeptide having at least 95% identity or at least 98% identity to the modified IgG3 heavy chain constant domain of SEQ ID NO:2.
7. The engineered antibody according to any of items 3-6, wherein said IgG3 region comprises the modified IgG3 heavy chain constant domain of SEQ ID NO:2.
8. The engineered antibody according to item 1, wherein the Fc region is an IgG1 region.
9. The engineered antibody according to item 8, wherein said engineered antibody comprises an IgG1 constant domain engineered with IgG3 hinge, SEQ ID NO:3.
10. The engineered antibody according to any of items 1-9, wherein said viral spike protein is from a corona virus.
11. The engineered antibody according to any of items 1-10, wherein said viral spike protein is a SARS-Cov-2 spike protein.
12. The engineered antibody according to any of items 1-11, wherein said viral spike protein is selected from the group consisting of the Wuhan spike protein of SARS-Cov-2 (SEQ ID NO:4), the Delta (B1.617.2) spike protein of SARS-Cov-2 (SEQ ID NO:5) and the Omicron (B1.1529) spike protein of SARS-Cov-2 (SEQ ID NO:6).
13. The engineered antibody according to any of items 1-12, wherein said viral spike protein is the Wuhan spike protein of SARS-Cov-2 (SEQ ID NO:4).
14. The engineered antibody according to any of items 1-12, wherein said viral spike protein is the Delta (B1.617.2) spike protein of SARS-Cov-2 (SEQ ID NO:5).
15. The engineered antibody according to any of items 1-12, wherein said viral spike protein is the Omicron (B1.1529) spike protein of SARS-Cov-2 (SEQ ID NO:6).
16. The engineered antibody according to any of items 1-9, wherein said viral spike protein is from Orthomyxoviruses, paramyxoviruses, rhabdoviruses, bunyaviruses, arenaviruses or retroviruses, such as from HIV, Herpesvirus, Rubella or Filoviruses.
17. The engineered antibody according to any of items 1-16, wherein said IgG3 hinge comprises an amino acid sequence which has at least 90% identity, at least 95% identity, at least 98% identity or 100% identity to the amino acid sequence ELKTPLGDTTHTCPRCPEPSKCDTPPPCPRCPEPSKCDTPPPCPRCPEPSKCDTPPPCPRCP (SEQ ID NO:7).
18. The engineered antibody according to any of items 1-17, wherein said IgG3 hinge comprises an amino acid sequence which is at least 90% identity, at least 95% identity, at least 98% identity or 100% identity to the amino acid sequence X-(Y)n, wherein
   X is ELKTPLGDTTHTCPRCP (SEQ ID NO:8),
   Y is EPSKCDTPPPCPRCP (SEQ ID NO:9), and
   n is an integer in the range from 2 to 8, preferably in the range from 2 to 5, more preferably in the range from 2 to 4.
19. The engineered antibody according to item 18, wherein n is 2.
20. The engineered antibody according to item 18, wherein n is 3.
21. The engineered antibody according to item 18, wherein n is 4.
22. The engineered antibody according to any of items 1-21, which is an isolated antibody.
23. The engineered antibody according to any of items 1-22, which promotes opsonisation.
24. The engineered antibody according to any of items 1-22, which does not bind to neutralising epitopes.
25. The engineered antibody according to any of items 1-22, wherein said engineered antibody is selected from the group consisting of Ab11, Ab36, Ab57, Ab59, Ab66, Ab77, Ab81 and Ab94.
26. The engineered antibody according to any of items 1-25, wherein said engineered antibody is selected from the group consisting of Ab11, Ab36, Ab57, Ab59, Ab66 and Ab77.
27. The engineered antibody according to any of items 1-26, wherein the heavy variable domain is SEQ ID NO:10 and the light variable domain is SEQ ID NO:11.
28. The engineered antibody according to any of items 25-27, which is Ab11.
29. The engineered antibody according to any of items 1-26, wherein the heavy variable domain is SEQ ID NO:12 and the light variable domain is SEQ ID NO:13.
30. The engineered antibody according to any of items 25, 26 or 29, which is Ab36.
31. The engineered antibody according to any of items 1-26, wherein the heavy variable domain is SEQ ID NO:14 and the light variable domain is SEQ ID NO:15.
32. The engineered antibody according item 25, 26 or 31, which is Ab57.
33. The engineered antibody according to any of items 1-26, wherein the heavy variable domain is SEQ ID NO:16 and the light variable domain is SEQ ID NO:17.
34. The engineered antibody according to item 25, 26 or 35, which is Ab59.
35. The engineered antibody according to any of items 1-26, wherein the heavy variable domain is SEQ ID NO:18 and the light variable domain is SEQ ID NO:19.
36. The engineered antibody according to item 25, 26 or 35, which is Ab66.
37. The engineered antibody according to any of items 1-26, wherein the heavy variable domain is SEQ ID NO:20 and the light variable domain is SEQ ID NO:21.
38. The engineered antibody according to item 25 or 37, which is Ab77.
39. The engineered antibody according to any of items 1-25, wherein the heavy variable domain is SEQ ID NO:22 and the light variable domain is SEQ ID NO:23.
40. The engineered antibody according to item 25 or 39, which is Ab81.
41. The engineered antibody according to any of items 1-25, wherein the heavy variable domain is SEQ ID NO:24 and the light variable domain is SEQ ID NO:25.
42. The engineered antibody according to item 25 or 41, which is Ab94.
43. A composition comprising at least two different engineered antibodies as defined in any of items 1-42.
44. The composition according to item 43, comprising at least three, at least four, at least five, at least eight, or at least ten different engineered antibodies as defined in any of items 1-42.
45. The composition according to any of items 43-44, comprising from 3 to 15, from 3 to 12, from 3 to 9, from 5 to 15, from 5 to 10, or from 6 to 10 different engineered antibodies as defined in any of items 1-42.
46. The composition according to any of items 43-44, wherein said at least two, at least three, at least four, at least five, at least eight, or at least ten different engineered antibodies have different viral spike protein binding parts.
47. The composition according to item 45, wherein said from 3 to 15, from 3 to 12, from 3 to 9, from 5 to 15, from 5 to 10, or from 6 to 10 different engineered antibodies have different viral spike protein binding parts.
48. The composition according to any of items 43-47, which is a pharmaceutical composition.
49. The composition according to any of items 43-48, additionally comprising a pharmaceutically acceptable excipient.
50. The composition according to any of items 43-49, which is sterile.
51. A nucleic acid molecule encoding a protein comprising the engineered antibody as defined in any of items 1-42.
52. The nucleic acid molecule according to item 51, which encodes the engineered antibody as defined in any of items 1-42.
53. A cell comprising the nucleic acid according to item 51 or 52.
54. Use of the engineered antibody as defined in any of items 1-42, for preventing, treating or alleviating a viral infection caused by a virus comprising said spike protein.
55. Use of the engineered antibody as defined in any of items 1-42, for Fc-mediated phagocytosis of a virus comprising said spike protein.
56. Use of the composition as defined in any of items 43-50, for preventing, treating or alleviating a viral infection caused by a virus comprising said spike protein.
57. Use of the composition as defined in any of items 43-50, for activating the complement pathway.
58. Use of the composition as defined in any of items 34-41, for Fc-mediated phagocytosis of a virus comprising said spike protein.
59. The use according to any of items 54-58, wherein said spike protein is from a corona virus.
60. The use according to any of items 54-59, wherein said viral spike protein is a SARS-Cov-2 spike protein.
61. The use according to any of items 54-60, wherein said viral spike protein is selected from the group consisting of the Wuhan spike protein of SARS-Cov-2 (SEQ ID NO:4), the Delta (B1.617.2) spike protein of SARS-Cov-2 (SEQ ID NO:5) and the Omicron (B1.1529) spike protein of SARS-Cov-2 (SEQ ID NO:6).
62. The use according to any of items 54-61, wherein said viral spike protein is the Wuhan spike protein of SARS-Cov-2 (SEQ ID NO:4).
63. The use according to any of items 54-61, wherein said viral spike protein is the Delta (B1.617.2) spike protein of SARS-Cov-2 (SEQ ID NO:5).
64. The use according to any of items 54-61, wherein said viral spike protein is the Omicron (B1.1529) spike protein of SARS-Cov-2 (SEQ ID NO:6).
65. The use according to any of items 54-58, wherein said viral spike protein is from Orthomyxoviruses, paramyxoviruses, rhabdoviruses, bunyaviruses, arenaviruses or retroviruses, such as from HIV, Herpesvirus, Rubella or Filoviruses.
66. A method for preparing an engineered antibody as defined in any of items 1-42, comprising the steps:
   - immunization of a host organism with said viral spike protein or a protein comprising said viral spike protein,
   - isolation of at least one viral spike protein reactive B-cell to isolate an IgG1 antibody binding to said viral spike protein,
   - preparing at least one recombinant DNA molecule which encodes a modified version of said IgG1 antibody comprising the replacing of the IgG1 subclass specific domain of said isolated IgG1 antibody with the IgG3 subclass specific domain,
   - expressing said at least one recombinant DNA molecule in a suitable host cell,
   to obtain said engineered antibody.
67. A method for preparing an engineered antibody as defined in any of items 1-42, comprising the steps:
   - immunization of a host organism with said viral spike protein or a protein comprising said viral spike protein,
   - isolation of at least one viral spike protein reactive B-cell to isolate an IgG1 antibody binding to said viral spike protein,
   - preparing at least one recombinant DNA molecule which encodes a modified version of said IgG1 antibody, said modification comprising the replacing of the IgG1 hinge of said isolated IgG1 antibody with an IgG3 hinge,
   - expressing said at least one recombinant DNA molecule in a suitable host cell,
   to obtain said engineered antibody.
68. The method according to item 67, wherein said IgG3 hinge comprises an amino acid sequence which has at least 90% identity, at least 95% identity, at least 98% identity or 100% identity to the amino acid sequence ELKTPLGDTTHTCPRCPEPSKCDTPPPCPRCPEPSKCDTPPPCPRCPEPSKCDTPPPCPRCP (SEQ ID NO:7).
69. The method according to item 67, wherein said IgG3 hinge comprises an amino acid sequence which is at least 90% identity, at least 95% identity, at least 98% identity or 100% identity to the amino acid sequence X-(Y)n, wherein
   X is ELKTPLGDTTHTCPRCP (SEQ ID NO:8),
   Y is EPSKCDTPPPCPRCP (SEQ ID NO:9), and
   n is an integer in the range from 2 to 8, preferably in the range from 2 to 5, more preferably in the range from 2 to 4.
70. The method according to item 69, wherein n is 2, 3 or 4.
71. The method according to any of items 66-70, wherein said spike protein is from a corona virus.
72. The method according to any of items 66-71, wherein said viral spike protein is a SARS-Cov-2 spike protein.
73. The method according to any of items 66-72, wherein said viral spike protein is selected from the group consisting of the Wuhan spike protein of SARS-Cov-2 (SEQ ID NO:4), the Delta (B1.617.2) spike protein of SARS-Cov-2 (SEQ ID NO:5) and the Omicron (B1.1529) spike protein of SARS-Cov-2 (SEQ ID NO:6).

Compositions and formulations in accordance with the present invention are prepared in accordance with known standards for the preparation of pharmaceutical compositions and formulations. For instance, the compositions and formulations are prepared in a way that they can be stored and administered appropriately, e.g. by using pharmaceutically acceptable components such as carriers, excipients or stabilizers. Such pharmaceutically acceptable components are not toxic in the amounts used when administering the pharmaceutical composition or formulation to a patient. The pharmaceutical acceptable components added to the pharmaceutical compositions or formulations may depend on the chemical nature of the inhibitor and targeting agent present in the composition or formulation (depend on whether the targeting agent is e.g. an antibody or fragment thereof or a cell expressing a chimeric antigen receptor), the particular intended use of the pharmaceutical compositions and the route of administration.

In a preferred embodiment in accordance with the invention, the composition or formulation is suitable for administration to humans, preferably the formulation is sterile and/or non-pyrogenic.

### Definition of some terms.

Unless otherwise defined below, the terms used in the present invention shall be understood in accordance with the common meaning known to the person skilled in the art.

The term "antibody" (e.g. a monoclonal antibody) as described herein shall be understood in accordance with the common meaning known to the person skilled in the art. The antibody may be a derivatized antibody or be linked to a different molecule. For example, molecules that may be linked to the antibody are other proteins (e.g. other antibodies), a molecular label (e.g. a fluorescent, luminescent, colored or radioactive molecule), a pharmaceutical and/or a toxic agent. The antibody or antigen-binding portion may be linked directly (e.g. in form of a fusion between two proteins), or via a linker molecule (e.g. any suitable type of chemical linker known in the art).

The term "isolated antibody" as described herein is to be understood as an antibody which is recovered or purified to some extent. In an embodiment that extent means it is free from a multitude of antibodies having a different structure such as more than 25 antibodies. In another embodiment that extent means it is free from different antibodies not binding to the spike protein. The term "engineered antibody" as described herein shall be understood in accordance with the common meaning known to the person skilled in the art. Engineered antibodies are novel recombinant antibody molecules with improved antigen specificities and effector functions, which are typically produced by the recombinant DNA and protein engineering technologies.

As used herein, the term "IgG3 hinge" shall be understood to mean the hinge region from the IgG3 class of antibodies, including variants thereof. Non-limiting examples of IgG3 hinges are peptides having an amino acid sequence which has at least 90% identity, at least 95% identity, at least 98% identity or 100% identity to the amino acid sequence ELKTPLGDTTHTCPRCPEPSKCDTPPPCPRCPEPSKCDTPPPCPRCPEPSKCDTPPPCPRCP (SEQ ID NO:7). Other examples of IgG3 hinges have an amino acid sequence which is at least 90% identity, at least 95% identity, at least 98% identity or 100% identity to the amino acid sequence X-(Y)n, wherein X is ELKTPLGDTTHTCPRCP (SEQ ID NO:8), Y is EPSKCDTPPPCPRCP (SEQ ID NO:9), and n is an integer in the range from 2 to 8, preferably in the range from 2 to 5, more preferably in the range from 2 to 4.

The term "viral spike protein" as used herein refers to the spike like proteins situated on the outside of many viruses. In contrast to the membrane protein and the envelope protein that are primarily involved in virus assembly, the spike protein plays a crucial role in penetrating host cells and initiating infection.

The term "bind" or "binding" in relation to antibodies as used herein refers to the forming of a complex with a molecule that is to be bound, e.g. the spike protein of SARS-Cov-2. Binding typically occurs non-covalently by intermolecular forces, such as ionic bonds, hydrogen bonds and Van der Waals forces and is typically reversible. Various methods and assays to determine binding capability are known in the art. Binding is usually a binding with high affinity, wherein the affinity as measured in KD values is preferably less than 1 mM, more preferably less than 100 nM, even more preferably less than 10 nM, even more preferably less than 1 nM, even more preferably less than 100 pM, even more preferably less than 10 pM, and even more preferably less than 1 pM.

As used herein, each occurrence of terms such as "comprising" or "comprises" may optionally be substituted with "consisting of" or "consists of'.

Terms such as "treating", "preventing" or "alleviating" according to the present invention refer to a therapeutic treatment. An assessment of whether or not a therapeutic treatment works can, for instance, be made by assessing whether the treatment prevents or inhibits viral infection in the treated patient or patients. Preferably, the prevention or inhibition is statistically significant as assessed by appropriate statistical tests which are known in the art. Inhibition or prevention of viral infection may be assessed by comparing the degree of viral infection in a group of subjects infected or exposed to the virus which are administered an engineered antibody according to the present invention to a control group of subjects that are infected or exposed but who is administered placebo. Alternatively, it may be assessed by comparing a group of infected patients that receive a standard antiviral treatment of the art plus a treatment according to the invention with a control group of patients that only receive a standard antiviral treatment of the art. Such studies for assessing the inhibition or prevention of viral infection are designed in accordance with accepted standards for clinical studies, e.g. double-blinded, randomized studies with sufficient statistical power.

As used herein, the term "pharmaceutically acceptable" means being approved by a regulatory agency of the Federal or a state government or listed in the U.S. Pharmacopia, European Pharmacopia or other generally recognized pharmacopia for use in mammals, and more particularly in humans. Pharmaceutically acceptable carriers include, but are not limited to, saline, buffered saline, dextrose, water, glycerol, sterile isotonic aqueous buffer, and combinations thereof.

The sequences referred to in the present description are the following :
SEQ ID NO:1 (lgG3 Heavy chain constant domain) 
ASTKGPSVFPLAPCSRSTSGGTAALGCLVKDYFPEPVTVSWNSGALTSGVHTFPAVLQSSGLYSLSSVVTVPSSSL
GTQTYTCNVNHKPSNTKVDKRVELKTPLGDTTHTCPRCPEPKSCDTPPPCPRCPEPKSCDTPPPCPRCPEPKSCD
TPPPCPRCPAPELLGGPSVFLFPPKPKDTLMISRTPEVTCVVVDVSHEDPEVQFKWYVDGVEVHNAKTKPREEQ
FNSTFRVVSVLTVLHQDWLNGKEYKCKVSNKALPAPIEKTISKTKGQPREPQVYTLPPSREEMTKNQVSLTCLVK
GFYPSDIAVEWESSGQPENNYNTTPPMLDSDGSFFLYSKLTVDKSRWQQGNIFSCSVMHEALHNRFTQKSLSLS
PGK
SEQ ID NO:2 (Modified IgG3 Heavy chain constant domain) 
ASTKGPSVFPLAPCSRSTSGGTAALGCLVKDYFPEPVTVSWNSGALTSGVHTFPAVLQSSGLYSLSSVVTVPSSSL
GTQTYTCNVNHKPSNTKVDKRVELKTPLGDTTHTCPRCPEPKSCDTPPPCPRCPEPKSCDTPPPCPRCPEPKSCD
TPPPCPRCPAPELLGGPSVFLFPPKPKDTLMISRTPEVTCVVVDVSHEDPEVQFKWYVDGVEVHNAKTKPREEQ
FNSTFRVVSVLTVLHQDWLNGKEYKCKVSNKALPAPIEKTISKTKGQPREPQVYTLPPSREEMTKNQVSLTCLVK
 
GFYPSDIAVEWESSGQPENNYKTTPPVLDSDGSFFLYSKLTVDKSRWQQGNIFSCSVMHEALHNHFTQKSLSLS
PGK
SEQ ID NO:3 (IgG1 constant domain engineered with IgG3 hinge) 
ASTKGPSVFPLAPSSKSTSGGTAALGCLVKDYFPEPVTVSWNSGALTSGVHTFPAVLQSSGLYSLSSVVTVPSSSL
GTQTYICNVNHKPSNTKVDKKVELKTPLGDTTHTCPRCPEPKSCDTPPPCPRCPEPKSCDTPPPCPRCPEPKSCD
TPPPCPRCPAPELLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEALHNHYTQKSLSL
SPGK
SEQ ID NO:4 (Wuhan Sars-CoV-2 spike protein) 
MFVFLVLLPLVSSQCVNLTTRTQLPPAYTNSFTRGVYYPDKVFRSSVLHSTQDLFLPFFSNVTWFHAISGTNGTK
RFDNPVLPFNDGVYFASTEKSNIIRGWIFGTTLDSKTQSLLIVNNATNVVIKVCEFQFCNDPFLGVYHKNNKSW
MESEFRVYSSANNCTFEYVSQPFLMDLEGKQGNFKNLREFVFKNIDGYFKIYSKHTPINLVRDLPQGFSALEPLV
DLPIGINITRFQTLLALHRSYLTPGDSSSGWTAGAAAYYVGYLQPRTFLLKYNENGTITDAVDCALDPLSETKCTLK
SFTVEKGIYQTSNFRVQPTESIVRFPNITNLCPFGEVFNATRFASVYAWNRKRISNCVADYSVLYNSASFSTFKCY
GVSPTKLNDLCFTNVYADSFVIRGDEVRQIAPGQTGKIADYNYKLPDDFTGCVIAWNSNNLDSKVGGNYNYLYR
LFRKSNLKPFERDISTEIYQAGSTPCNGVEGFNCYFPLQSYGFQPTYGVGYQPYRVVVLSFELLHAPATVCGPKKS
TNLVKNKCVNFNFNGLTGTGVLTESNKKFLPFQQFGRDIDDTTDAVRDPQTLEILDITPCSFGGVSVITPGTNTS
NQVAVLYQGVNCTEVPVAIHADQLTPTWRVYSTGSNVFQTRAGCLIGAEHVNNSYECDIPIGAGICASYQTQT
NSHRRARSVASQSIIAYTMSLGAENSVAYSNNSIAIPINFTISVTTEILPVSMTKTSVDCTMYICGDSTECSNLLLQY
GSFCTQLNRALTGIAVEQDKNTQEVFAQVKQIYKTPPIKDFGGFNFSQILPDPSKPSKRSFIEDLLFNKVTLADAG
FIKQYGDCLGDIAARDLICAQKFNGLTVLPPLLTDEMIAQYTSALLAGTITSGWTFGAGAALQIPFAMQMAYRF
NGIGVTQNVLYENQKLIANQFNSAIGKIQDSLSSTASALGKLQDVVNQNAQALNTLVKQLSSNFGAISSVLNDIL
ARLDKVEAEVQIDRLITGRLQSLQTYVTQQLIRAAEIRASANLAATKMSECVLGQSKRVDFCGKGYHLMSFPQSA
PHGVVFLHVTYVPAQEKNFTTAPAICHDGKAHFPREGVFVSNGTHWFVTQRNFYEPQIITTHNTFVSGNCDVVI
GIVNNTVYDPLQPELDSFKEELDKYFKNHTSPDVDLGDISGINASVVNIQKEIDRLNEVAKNLNESLIDLQELGKYE
QYIKWPWYIWLGFIAGLIAIVMVTIMLCCMTSCCSCLKGCCSCGSCCKFDEDDSEPVLKGVKLHYT
SEQ ID NO:5 (Delta (B1.617.2) spike Sars-CoV-2 variant) 
MFVFLVLLPLVSSQCVNLRTRTQLPPAYTNSFTRGVYYPDKVFRSSVLHSTQDLFLPFFSNVTWFHAIHVSGTNG
TTRFDNPVLPFNDGVYFASTEKSNIIRGWIFGTTLDSKTQSLLIVNNATNVVIKVCEFQFCNDPFLGVYYHKNNKS
 
WMESGVYSSANNCTFEYVSQPFLMDLEGKQGNFKNLREFVFKNIDGYFKIYSKHTPINLVRDLPQGFSALEPLV
DLPIGINITRFQTLLALHRSYLTPGDSSSGWTAGAAAYYVGYLQPRTFLLKYNENGTITDAVDCALDPLSETKCTLK
SFTVEKGIYQTSNFRVQPTESIVRFPNITNLCPFGEVFNATRFASVYAWNRKRISNCVADYSVLYNSASFSTFKCY
GVSPTKLNDLCFTNVYADSFVIRGDEVRQIAPGQTGKIADYNYKLPDDFTGCVIAWNSNNLDSKVGGNYNYRY
RLFRKSNLKPFERDISTEIYQAGSKPCNGVEGFNCYFPLQSYGFQPTNGVGYQPYRVVVLSFELLHAPATVCGPK
KSTNLVKNKCVNFNFNGLTGTGVLTESNKKFLPFQQFGRDIADTTDAVRDPQTLEILDITPCSFGGVSVITPGTNT
SNQVAVLYQGVNCTEVPVAIHADQLTPTWRVYSTGSNVFQTRAGCLIGAEHVNNSYECDIPIGAGICASYQTQT
NSRRRARSVASQSIIAYTMSLGAENSVAYSNNSIAIPTNFTISVTTEILPVSMTKTSVDCTMYICGDSTECSNLLLQ
YGSFCTQLNRALTGIAVEQDKNTQEVFAQVKQIYKTPPIKDFGGFNFSQILPDPSKPSKRSFIEDLLFNKVTLADA
GFIKQYGDCLGDIAARDLICAQKFNGLTVLPPLLTDEMIAQYTSALLAGTITSGWTFGAGAALQIPFAMQMAYR
FNGIGVTQNVLYENQKLIANQFNSAIGKIQDSLSSTASALGKLQNVVNQNAQALNTLVKQLSSNFGAISSVLNDI
LSRLDKVEAEVQIDRLITGRLQSLQTYVTQQLIRAAEIRASANLAATKMSECVLGQSKRVDFCGKGYHLMSFPQS
APHGVVFLHVTYVPAQEKNFTTAPAICHDGKAHFPREGVFVSNGTHWFVTQRNFYEPQIITTDNTFVSGNCDV
VIGIVNNTVYDPLQPELDSFKEELDKYFKNHTSPDVDLGDISGINASVVNIQKEIDRLNEVAKNLNESLIDLQELGK
YEQYIKWPWYIWLGFIAGLIAIVMVTIMLCCMTSCCSCLKGCCSCGSCCKFDEDDSEPVLKGVKLHYT
SEQ ID NO:6 (Omicron (B1.1529) spike Sars-CoV-2 variant) 
MFVFLVLLPLVSSQCVNLTTRTQLPPAYTNSFTRGVYYPDKVFRSSVLHSTQDLFLPFFSNVTWFHVISGTNGTK
RFDNPVLPFNDGVYFASIEKSNIIRGWIFGTTLDSKTQSLLIVNNATNVVIKVCEFQFCNDPFLDHKNNKSWMES
EFRVYSSANNCTFEYVSQPFLMDLEGKQGNFKNLREFVFKNIDGYFKIYSKHTPIIVREPEDLPQGFSALEPLVDLP
IGINITRFQTLLALHRSYLTPGDSSSGWTAGAAAYYVGYLQPRTFLLKYNENGTITDAVDCALDPLSETKCTLKSFT
VEKGIYQTSNFRVQPTESIVRFPNITNLCPFDEVFNATRFASVYAWNRKRISNCVADYSVLYNLAPFFTFKCYGVS
PTKLNDLCFTNVYADSFVIRGDEVRQIAPGQTGNIADYNYKLPDDFTGCVIAWNSNKLDSKVSGNYNYLYRLFR
KSNLKPFERDISTEIYQAGNKPCNGVAGFNCYFPLRSYSFRPTYGVGHQPYRVVVLSFELLHAPATVCGPKKSTNL
VKNKCVNFNFNGLKGTGVLTESNKKFLPFQQFGRDIADTTDAVRDPQTLEILDITPCSFGGVSVITPGTNTSNQV
AVLYQGVNCTEVPVAIHADQLTPTWRVYSTGSNVFQTRAGCLIGAEYVNNSYECDIPIGAGICASYQTQTKSHR
RARSVASQSIIAYTMSLGAENSVAYSNNSIAIPTNFTISVTTEILPVSMTKTSVDCTMYICGDSTECSNLLLQYGSFC
TQLKRALTGIAVEQDKNTQEVFAQVKQIYKTPPIKYFGGFNFSQILPDPSKPSKRSFIEDLLFNKVTLADAGFIKQY
GDCLGDIAARDLICAQKFKGLTVLPPLLTDEMIAQYTSALLAGTITSGWTFGAGAALQIPFAMQMAYRFNGIGV
TQNVLYENQKLIANQFNSAIGKIQDSLSSTASALGKLQDVVNHNAQALNTLVKQLSSKFGAISSVLNDIFSRLDKV
EAEVQIDRLITGRLQSLQTYVTQQLIRAAEIRASANLAATKMSECVLGQSKRVDFCGKGYHLMSFPQSAPHGVV
FLHVTYVPAQEKNFTTAPAICHDGKAHFPREGVFVSNGTHWFVTQRNFYEPQIITTDNTFVSGNCDVVIGIVNN
 
TVYDPLQPELDSFKEELDKYFKNHTSPDVDLGDISGINASVVNIQKEIDRLNEVAKNLNESLIDLQELGKYEQYIK
WPWYIWLGFIAGLIAIVMVTIMLCCMTSCCSCLKGCCSCGSCCKFDEDDSEPVLKGVKLHYT
SEQ ID NO:7 (lgG3 hinge region)
   ELKTPLGDTTHTCPRCPEPKSCDTPPPCPRCPEPKSCDTPPPCPRCPEPKSCDTPPPCPRCP
SEQ ID NO:8 (IgG3 hinge part)
   ELKTPLGDTTHTCPRCP
SEQ ID NO:9 (IgG3 hinge part)
   EPSKCDTPPPCPRCP
SEQ ID NO: 10 (Clone 11 heavy variable domain) 
QVTLKESGPVLVKPTETLTLTCTVSGFSLSNAKMGVSWIRQPPGKALEWLAHIFSNDEKSYSTSLKSSLTISKDTSK
SQVVLTMTNMDPVDTATYYCARLLWFGGNYFDYWGQGTLVTVSS
SEQ ID NO: 11 (Clone 11 light variable domain) 
SYELTQPLSVSVALGQTARITCGGNNIGSKMHWYQQKPGQAPVLVIYRDSNRPSGIPERFSGSNSGNTATLTISR
AQAGDEADYYCQVWDSSTVVFGGGTKLTVL
SEQ ID NO: 12 (Clone 36 heavy variable domain) 
QVQLVQSGAEVKKPGASVKVSCKASGYTFTSHAVHWVRQAPGQRLEWMGWINAGNGNTKYSQKFQGRVTI
TRDTSASTAYMELSSLRSEDTAVYYCARDPVLRYFDWTTPYYFDYWGQGTLVTVSS
SEQ ID NO: 13 (Clone 36 light variable domain) 
EIVLTQSPGTLSLSPGERATLSCRASQSFSSSYLAWYQQKPGQAPRLLIYGASSRATGIPDRFSGSGSGTDFTLTIS
RLEPEDFAVYYCQHYGSSPLTFGGGTKVEIK
SEQ ID NO: 14 (Clone 57 heavy variable domain) 
QVTLRESGPALVRPTQTLTLTCTFSGFSLSTSGMCVSWIRQPPGKALEWLARIDWDDDKYYSTSLRTRLTISKDTS
KNQVVLTMTNMDPVDTATYYCARMTVTTAFDIWGQGTMVTVSS
SEQ ID NO: 15 (Clone 57 light variable domain) 
DIQMTQSPSSLSASVGDRVTITCRASQSISSYLNWYQQKPGKAPKLLIYAASSLQSGVPSRFSGSGSGTDFTLTISS
LQPEDFATYYCQQSYSTPRTFGQGTKVEIK
SEQ ID NO: 16 (Clone 59 heavy variable domain) 
QVQLVQSGAEVKKPGASVKVSCKASGYTFTSYDINWVRQATGQGLEWMGWMNPNSGNTAYAQKFQGRVT
MTRNTSISTAYMELSSLRSEDTAVYYCARGGRYCSSTSCYSGVPNDYWGQGTLVTVSS
SEQ ID NO: 17 (Clone 59 light variable domain) 
DIQLTQSPSFLSASVGDRVTITCRASQGVSSYLAWYQQKPGKAPKLLIYPASTLQSGVPSRFSGSGSGTEFTLTISS
LQPEDFATYYCQQLNSYPLTFGGGTKVEIK
SEQ ID NO: 18 (Clone 66 heavy variable domain) 
EVQLVESGGGLVKPGGSLRLSCAASGFTFSSYSMNWVRQAPGKGLEWVSSISSSSSYIYYADSVKGRFTISRDNA
KNSLYLQMNSLRAEDTAVYYCARRSFVSSSTDDYYYYYDMDVWGQGTTVTVSS
SEQ ID NO: 19 (Clone 66 light variable domain) 
SYELTQPPSVSVSPGQTARITCSGDALPKQYAYWYQQKPGQAPVLVIYKDSERPSGIPERFSGSSSGTTVTLTISG
VQAEDEADYYCQSADSSGTYVVFAGGTKLTVL
SEQ ID NO: 20 (Clone 77 heavy variable domain) 
QVQLVESGGGVVQPGRSLRLSCAASGFTFSSYDMHWVRQAPGKGLEWVALISYDGSNTYYAD

SVKGRFTISRDNSKNTLYLQMNSLRAEDTAVFYCAKTIYSYALKPNYFDYWGQGTLVTVSS
SEQ ID NO: 21 (Clone 77 light variable domain) 
DIQMTQSPSSLSASVGDRVTITCQASQDISNYLNWYQQKPGKAPKLLIYDASNLET

GVPSRFSGSGSGTDFTFTISSLQPEDIATYYCQQYDNLPPTFGGGTKVGIK
SEQ ID NO: 22 (Clone 81 heavy variable domain) 
QVQLVQSGAEVKKPGASVKVSCKASGYTFTSYGISWVRQAPGQGLEWMGWISAYNGNTNYA

QKVQGRVTMTRDTSTSTAYMELRSLRSDDTAVYYCARDGIAVAEDWFDPWGQGTLVTVSS
SEQ ID NO: 23 (Clone 81 light variable domain) 
SYELTQPPSVSVAPGKTARITCGENNIGSKSVHWYQQKPGQAPVLVIYYDSDRP

SGIPERFSGSNSGNTATLTISRVEAGDEADYYCQVWDGSSDHVVFGGGTKLTVL
SEQ ID NO: 24 (Clone 94 heavy variable domain) 
QVQLQESGPRLVKPSGTLSLTCAVSGGSISSSNWWTWVRQPPGKGLEWIGEIYHSGSTNYTPS

LKSRVTISVDKSKNQFSLRLNSVTAADTAVYYCARGWSSSWYGLDYWGQGTLVTVSS
SEQ ID NO: 25 (Clone 94 light variable domain)

QSALTQPASVSGSPGQSITISCTGTSSDVGSYNLVSWYQQHPGKAPKLVIYEATKRPSGVSNRFSGSKSGNTASL
TISGLQAEDEADYYCYSYAGSSTWVFGGGTKLTVL

### Examples

### Materials and Methods

### IgG3 plasmid generation, production, and sequencing

The anti-spike monoclonal antibodies used in this work were made from spike-reactive B-cells from convalescent patients who were infected 6 weeks earlier with SARS-CoV-2 (verified in the clinic). Briefly, the V(D)J coding sequences from spike-reactive B cells were generated using 10X Genomics. The variable regions were cloned into pTwist IgG1 vectors (Twist Biosciences) containing the IgG1 constant heavy domain. The plasmid containing the constant heavy domain gene for IgG3 was produced previously (Izadi et al 2022, unpublished). The sequence of the recombinant vectors for the light chain and heavy chain plasmids including constant domains are shown in Supplemental material document 1. Primers were designed so the insert (IgG3 heavy chain constant domain) contained overhangs that were complementary to the vector and homologous recombination could occur. This allowed the exchange of IgG1 constant domain into an IgG3 constant domain, leaving the variable region intact. The exchanging of the heavy chain constant domains was done by PCR of the IgG1 vector backbone and the IgG3 constant region which would replace the IgG1-specific domain. Subsequent HIFI-DNA assembly (NEB, biolAbs) would mediate the homologous recombination process. PCR products were loaded on agarose gels to assess correct amplification based on size. The bands were excised and purified by QIAGEN PCR clean-up kit according to manufacturer's instructions. Competent *E. coli* supplied in the kit was transformed with the newly assembled IgG3 plasmids and plated on agar plates containing ampicillin. The purified IgG3 plasmids from transformed colonies were verified by sequencing.

### Cell culture and antibody production

Expi293F suspension cells were purchased from Gibco (ThermoFisher) and routinely cultured in 125 ml Erlenmeyer flasks (Nalgene) in 30 ml Expi293 medium (Gibco) in an Eppendorf s41i shaker incubator at 37°C, 8 % CO₂, 120 rpm. Cells were routinely passed and split to a density of 0.5 × 10⁶ cells/ml every 3 to 4 days. The day before transfection, the cells were seeded at a density of 2 × 10⁶ cells/ml. The next day, cells were seeded at 7.5 × 10⁷ cells in 25.5 ml Expi293 medium. Transfection with heavy and light chain plasmids was carried out using the Expifectamine293 kit (Gibco) according to the manufacturer's instructions. Briefly, 20 µg of heavy and light chain plasmid, respectively, were mixed with 2.8 ml OptiMEM (Gibco) and 100 µl Expifectamine and incubated at room temperature for 15 minutes. Afterwards, the transfection mix was added to the Expi293F cells. The following day, 1.5 ml of enhancer 1 and 0.15 ml of enhancer 2 (both from the Expifectamine293 kit) were added and the cells cultured for another 72 hours.

The cells were removed from the cell culture medium by centrifugation (400 × g, 5 min) and the supernatant transferred to a new tube. In order to capture the IgGs from the medium, protein G sepharose 4 Fast Flow (Cytiva) was added to the medium and incubated end-over-end at room temperature for 2 hours. The beads were collected by running the medium bead mix through a gravity flow chromatography column (Biorad) and washed twice with 50 ml PBS. The antibodies were eluted using 5 ml HCl-glycine (0.1 M, pH 2.7). Tris (1 M, pH 8, 1 ml) was used to neutralize the pH. The buffer was exchanged to PBS using Amicon Ultra-15 centrifugal filters (Sigma) with a molecular cut-off of 30,000 Da. The concentration and quality of the purified antibodies was spectrophotometrically measured with the IgG setting of the Nanodrop (Denovix) as well as subjecting the antibodies to an SDS-PAGE and comparing the band intensity to a serial dilution of Xolair (Omalizumab).

### Generation of spike-coated beads

Spike-protein was generated by transfecting Expi293F cells with 40 µg plasmid containing the gene for the spike protein (CS/PP spike encoding a secretable version of the protein was used to allow purification from cell culture supernatants), donated previously to us by Dr. Florian Krammer's lab ⁵. 200 µg of spike protein was biotinylated according to the instructions of EZ-LinkTM Micro Sulfo-NHS-LCBiotinylation Kit (ThermoFisher). Then 500 µl (5×10⁷ million beads) of fluorescent (APC) streptavidin microsphere beads (1 µm, Bangs Laboratories) were conjugated with the biotinylated spike protein (200 µg) according to the manufacturer's instructions.

### Flow cytometry-based avidity measurements

The binding assays were performed in a 96-well plate which had been pre-coated with 200 µl of 2% BSA (in PBS) overnight at 4 °C. 100,000 spike coated beads were used in all wells, with antibody concentrations as the variable ranging between 0.02-0.2-2 µg per ml. The beads were opsonized at a volume of 100 µl in 1X PBS at 37 °C for 30 min on a shaking heat block. The wells were washed twice with PBS. To assess antibody binding to spike beads, 50 µl of (1:500 diluted) a Fab-specific fluorescent secondary antibody (#109-546-097, Jackson ImmunoResearch) was used to create a fluorescent signal. The secondary antibody was left to incubate with the spike-bead antibody complex at 37 °Cs for 30 minutes on a shaking heat block. 100 µl of PBS was added to the wells before analysis in the flow cytometer. The beads were analyzed using a Beckman Coulter Cytoflex flow cytometer, which acquired 20000 beads per sample. The data was processed using Flowjo. The gate for spike beads was set based on forward and side scatter. The gate for spike beads positive for antibodies was set based on reactivity to a non-reactive IgG control mAb. The data was later analyzed in Graphpad Prism using a nonlinear regression model: Hill slope was unconstrained, maximum value (Bmax) were set to 100% and K_{D} was constrained to be any value between 0-10000. The best fit value was presented in the Graph shown with the fitness of the model.

### SPR kinetic assay

To study binding kinetics to spike trimer, RBD or NTD we immobilized A High Capacity Amine Sensor chip (Bruker) was immobilized with Anti-human IgG (Fc) antibody (Cytiva BR-1008-39) at 25 µg/ml in 10 mM sodium acetate buffer pH 5 at flow rate 10 µl/min and contacting 300s. This was done in a MASS-16 biosensor instrument (Bruker). Running buffer PBS + 0,05%Tween20. The antibodies were diluted in PBS and injected over the surface for 90s at 10 µL/min. The running buffer was PBS with 0.01% Tween 20. The RBD-antigen used was produced in HEK293 acquired from Sinobiological (Beijing, China: RBD WT 40592-V08H). The spike trimer was produced as described above. The NTD-antigens were acquired from Sinobiological (Beijing, China: NTD 40591-V9H and NTD-Omicron 40591-V08H42). The RBD-antigen was diluted from 60 nM to 3.75 through serial halving dilutions. The NTD-antigen was in turn diluted from from 80 to 2.5 nM, by serial halving dilutions. The spike trimer was added at 40 nM. The antigens were injected at these concentrations and were allowed to interact with the sensor for 2 minutes, with flow rate 30 µl/min, followed by a dissociation for 6 minutes. After each cycle the surface was regenerated with 3M MgCl. All experiments with RBD and NTD were performed three times, and once with the spike trimer. The data was analyzed using Sierra Analyser software version 3.4.3 (Bruker) program to determine apparent dissociation constants (K_{D}).

### ELISA avidity measurements

For ELISA, 1 µg/ml of spike protein in PBS was immobilized onto ELISA High-bind plates (Sarstedt) overnight at 4°C. The wells were washed with PBST (1x PBS with 0.05 % Tween 20) 3 times and then blocked with 2% BSA/PBS for 1 hour at room temperature. After three washes, a serial dilution of primary antibody (anti-spike mAb) was added (100 µl of mAbs with a concentration of 0.08 up to 20 µg/ml) and incubated for 1 hour at room temperature. The wells were washed three times. A rabbit anti-human light chain-HRP secondary antibody (anti-kappa for clone 57, anti-lambda for clone 11 and 66) (Abcam ab202549 and ab200966) at a dilution of 1:5000 in PBS was added and left to incubate for 1 hour at room temperature. The wells were washed 3 more times with PBST. Finally, 100 µl developing reagent (20 ml sodium citrate pH 4.5 + 1 ml ABTS (2,2'-Azino-bis(3-ethylbenzothiazoline-6-sulfonic acid) diammonium salt, 0.2 g in 10 ml water, Sigma) + 0.4 ml 0.6 % H₂O₂) was added. OD450 was recorded after 15 - 30 minutes. Data was plotted with GraphPad Prism.

### C1q deposition assay

To study complement activation, we opsonized 500,000 spike beads with 0.1-1-10 µg/ml of mAbs with 1% antibody-depleted serum as a source for complement in a 96-well plate previously coated with 2% BSA. After 30 minutes of opsonization at 37 °Cs on a shaking heat block (300 RPM), the wells were washed 2 times with PBS before a FITC fluorescently labeled anti-C1q Ab was added (ab4223, Abcam) (1:250 dilution from stock, 50 µl). The anti-C1q Ab was left with the beads for 30 min at 37 °Cs on a shaking heat block (300 RPM). PBS was added so that the beads were resuspended in a final volume of 150 µl. The gate for spike beads was set based on forward and side scatter, and the gate for C1q deposition was set based on the results of the control-IgG1(Supp Fig. 5A). The data acquired were analyzed with Flowjo and plotted in GraphPad Prism. The EC₅₀-analysis was performed on GraphPad Prism using a non-linear regression model with the bottom constrained to 0 and the top value constrained to 100% for the % beads-C1q deposition analysis. For the MFI EC₅₀ analysis, the top was set to be shared and greater than 0.

### Flow cytometry-based phagocytosis assays

Phagocytosis experiments were done with the same batch of spike beads used in the binding assay. To assess internalization, the red-fluorescent beads were stained with pHrodo green (P35369, ThermoFischer), a pH sensitive dye using 5 µl of 10 mM of pHrodo green (ThermoFischer) in 95 µl of Na₂CO₃ (pH 9) for 30 min at 37 °Cs. Excess stain was washed away with PBS. THP-1 cells (Sigma-Aldrich) were cultured as described previously ⁵. In all experiments, 1×10⁵ THP-1 cells were used. In one set of experiments the ratio of cells to beads was 1:30 (Multiplicity of prey, MOP, 30) and the other it was 1:15 (MOP 15). For the MOP 30 experiments 3×10⁶ spike beads were opsonized with 10 µg/ml of antibodies in a volume of 100 µl of Sodium media (pH adjusted to 7.3 with NaOH; 5.6 mM glucose, 10.8 mM, 127 mM NaCl, KCI, 2.4 mM KH₂PO4, 1.6 mM MgSO4, 10 mM HEPES, 1.8 mM CaCl₂). For the MOP 15 experiments 1.5×10⁶ spike beads were opsonized with 5 µg/ml of mAbs. Both opsonizations were performed for 30 minutes at 37 °C on a shaking heat block (300 RPM) in a volume of 100 µl. During this incubation period, THP-1 cells were counted (using a Bürker chamber) and medium was exchanged from RPMI to Sodium medium. THP-1 cells (50 µl, 2×10⁶/ml) were added to each well and the cells were allowed to phagocytose beads for 30 minutes at 37 °C on a shaking heat block (300 RPM). The 96-well plate was then incubated on ice for 15 minutes and analyzed directly in a Beckman Coulter Cytoflex flow cytometer. A gate for THP-1 cells was set up based on their forward and side scatter (Supp Fig. 4A). The gate for internalization and association was set with a negative control of cells only (Fig. 3A). The analysis stopped after 5000 events were captured in the THP-1-gate. The data was analyzed in the program Flowjo by setting similar gates as described above. The Flowjo-processed data was further analyzed in GraphPad Prism and internalization and bead signal (APC-A) of the THP-1 gate were plotted to compare the different antibodies.

Monocytes were isolated from blood first by acquiring a PBMC layer by using a polymorphoprep (Abbot). Monocytes were purified from the PBMC layer through positive selection using CD14 Microbeads (Cat#130-050-201, Miltenyi Biotec) according to the manufacturer's instructions. Following isolation, monocytes were counted using a XN-350 hematology analyser (Sysmex). Neutrophils were in turn isolated with polymorphprep gradient according to the manufacturer's instructions and were counted with a Bürker chamber. Isolated cells were kept on ice for 1 hour after being resuspended in sodium media and adjusted to 2×10⁶ cells/ml. Donors had given written and oral consent to participate in this study and provided oral information on the purpose of the donation, which was used only to isolate and use the monocytes and neutrophils. This study used a previously approved ethical approval from the Swedish ethical review authority (2020/01747).

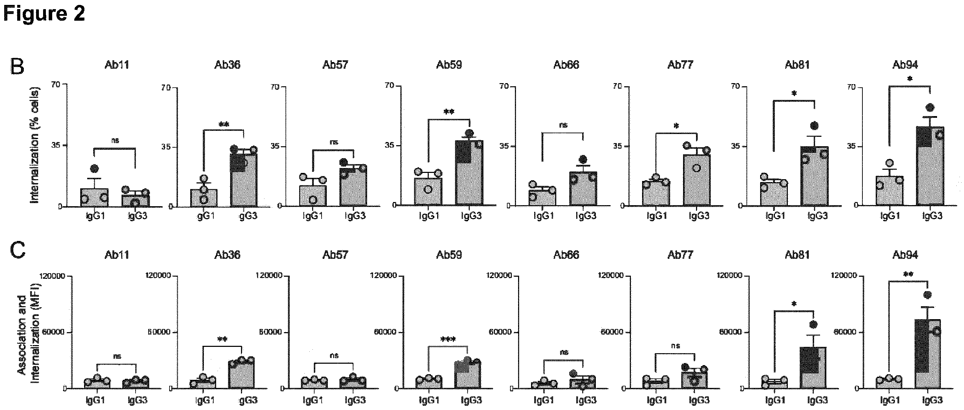

For monocyte phagocytosis experiments, spike beads were opsonized with 10 µg/ml of mAbs (IgG control mAb or OctomAb IgG3) but without any serum. Monocytes (1×10⁵cells) were added in a volume of 50 µl. The cells were left to phagocytose for 30 min at 37 °C on a shaking heat block (300 RPM). Following this, the cells were stained on ice for 20 minutes with a CD14 antibody (Brilliant Violet 421^{™} anti-human CD14 Antibody, Biolegend) diluted 1:50 to 3 µg/ml and added in a volume of 20 µl. The wells were analyzed in a Beckman Coulter Cytoflex Flow cytometer. Monocytes were gated using size and granularity (Supp. Fig. 2C). Further selection was done by gating for CD14 positive cells. The analysis was stopped after 3000 events were recorded in the CD14+ gate. The percentage of cells associated with spike beads (APC+) and internalized with beads (APC+, FITC+) (Supp. Fig. 2C) were plotted against MOP in Prism Graphpad.

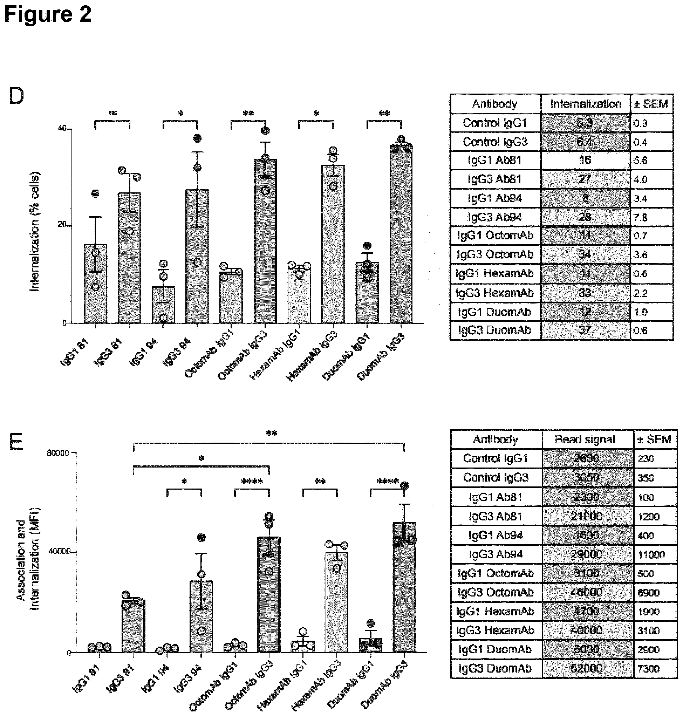

For the neutrophil experiments, the spike beads were opsonized with 10 µg/ml of mAbs with 1% antibody depleted serum (Pel-Freeze, Cat#34014-10) for 30 min at 37 °C on a shaking heat block (300 RPM) (100 µl final volume). To only study the effects of Fc-mediated phagocytosis, we heat-inactivated the serum at 56 °C for 1 hour. Both the heat-inactivated serum and normal human serum were added to the respective wells. Neutrophils (1×10⁵ cells) were added to each well with a volume of 50 µl (2×10⁶/ml). The cells were left to phagocytose for 30 min at 37 °C on a shaking heat block (300 RPM). The process of phagocytosis was stopped by putting the plate on ice. While on ice, the cells were stained with an anti-CD18 antibody (BV421 Mouse anti-Human CD18, BD Biosciences) diluted 1:100 to 2 µg/ml and 20 µl of this diluted antibody was added to the cells. The cells were stained on ice for 20 minutes before being analyzed in a Beckman Coulter Cytoflex flow cytometer. A gate was set for FSC-A and SSC-A to mark the neutrophils (Supp. Fig 2D). This population was further selected for by the CD18 marker (Supp Fig. 6B). The analysis stopped after 3000 events were captured in the CD18+ gate and the data was processed in the program for Flowjo with similar gates as described above. The percentage of cells with internalized beads and bead signal (APC) in the CD18 gate was used as metrics to compare the mAbs.

### IgG mAb aggregation quality control experiments

After production and purification of the mAbs we performed a quality control of the mAbs to test if aggregation could influence the results. We used clone 81 IgG1 and IgG3 as a sample issue. We centrifuged the samples at 16000 RCF for 10 minutes at room temperature. After centrifugation we removed the supernatant and sterile filtered it through a 0.2 micron syringe filter. We then compared the treated Ab81 IgG1 and IgG3 vs untreated variants of them to see if there was a difference in function. We performed phagocytosis experiments with THP-1 cells at MOP 30 as described above.

### Live imaging phagocytosis assay

### Cell preparation and spike bead opsonization

Neutrophils were isolated from 4 healthy donors through Polymorphprep as described above. The cells were then left on ice for 1 h in aliquots of 2×10⁶/ml per donor. Cells were seeded on an 8-well lbidi-plate (Cat#80827, Ibidi GmbH, Germany) at a density of 1×10⁵ cells/well for 1 hour with 5% CO₂ at 37°C. All Ibidi-plates were pre-coated with 3.3 µg/m! human fibronectin (F0895-1MG, Sigma) diluted in PBS. After the incubation, the cells were stained with Hoechst 1:20000 (ThermoFisher, Germany) and Alexa Fluor 594-conjugated wheat germ agglutinin (WGA) 1:333 diluted in sodium medium to a final volume of 300 µl/well. Excess stain was washed 3 times with 300 µl/well Sodium medium, before 250 µl/well of sodium medium was added.

Spike-biotinylated beads were pre-stained with pHrodo green prior to opsonization with 10 µg/ml antibodies (IgG3 OctomAb, IgG1 OctomAb, Ab94 IgG3, Control-IgG1) and 1% antibody-depleted serum (Pel-Freeze, Cat#34014-10). Beads were opsonized in a volume of 100 µl (containing 1×10⁶ beads) for 30 minutes at 37 °C shaking heat block. After opsonization, 5×10⁵ beads/well (50 µl) were added right before the initiation of live imaging to the previously seeded neutrophils.

### Live fluorescence imaging and data acquisition

Time-lapse images of phagocytosis were recorded every 10 minutes for 60 minutes using a Nikon N-SIM microscope. Images were acquired through fluorescent microscopy with a 20X Plan Apo λ objective (NA = 0.40) and Perfect Focus System (PFS). All images were taken with an ORCA-Flash 4.0 cCMOS camera (Hamamatsu Photonics K.K) at 8 positions per well. The filter cubes used were DAPI (Ex. 340 - 380 nm, Em. 435 - 485 nm), TxRed (Ex. 540 - 580 nm, Em. 600 - 660 nm), FITC (Ex. 465 - 495 nm, Em. 515 - 555 nm) and Cy5 (Ex. 625 - 650 nm, Em. Peak 670 nm). Samples were live imaged in an environmental chamber (Okolab) at 37°C with 5% CO₂. All image analysis was performed through the Nikon software General Analysis 3. Graphs and statistics were compiled in Microsoft Excel and Prism 9. The ET50 analysis was performed on Prism Graphpad, using a nonlinear regression model, hill-slope unconstrained and with bottom value constrained to 0 % and top value to 100%.

### SIM Image acquisition

After 60 minutes of phagocytosis, the sample was fixed in 4% paraformaldehyde with PBS at 37°C for 15 minutes. The wells were washed 3 times with sodium medium prior to SIM imaging. All SIM images were acquired using Nikon N-SIM microscope with a LU-NV laser unit (488/561/640 nm), CFI SR HP Apochromat TIRF 100X oil objective (NA = 1.49) and ORCA-Flash 4.0 cCMOS camera (Hamamatsu Photonics K.K). Filter cubes used were N-SIM 488 (Ex. 484 - 496 nm, Em. 500 - 545 nm), N-SIM 561 (Ex. 557 - 567 nm, Em. 579 - 640 nm) and N-SIM 640 (Ex. 629 - 645 nm, Em. 663 - 738 nm). Standard fluorescence microscopy was used to acquire images in the DAPI channel in the SIM system. For wide-field excitation a SPECTRA X light engine (Lumecore inc) was used. A Z-stack of 15 steps was collected for each image in all channels. SIM images were reconstructed using the Nikon SIM software through NIS Elements Ar (NIS-A 6D and N-SIM Analysis). Wide-field images were processed with NIS Element Clarify.ai and Denoise.ai to minimize blur from the fluorescens microscope and cropped from a 3x5 grid. All Z-stack images were compiled to a maximum intensity projection image before SIM- and fluorescens images were merged in the software NIS Elements.

### Animal experiment

Thirty seven- to ten-week old female K18 hACE2 (B6.Cg-Tg(K18-ACE2)2Prlmn/J) mice were split into 5 groups of 6 mice/group and antibodies were administered in one single dose, intraperitoneally. Eight hours after antibody administration, animals were inoculated intranasally with 10⁵ PFU of SARS-CoV-2 (Wuhan strain, isolate SARS-CoV-2/01/human/2020/SWE, sourced from the Swedish Health Authorities). The body weights and health status of the mice were recorded daily, and the animals were euthanized if they lost more than 20% of their body weights or showed a severe deterioration in health status. The infection proceeded for 10 days before the animals were euthanized. Blood, tissue and bronchoalveolar lavage were harvested and stored accordingly. All the animal experiments were performed under the approval of the regional animal experimental ethics committee in Stockholm (2020-2021). BAL-fluid analysis for viral titers were analyzed using qPCR⁵.

### Statistical analysis

Statistical analysis was performed in Graphpad Prism. For comparisons between subclasses in specific clones or between mixes a 2-tailed Welch's t-test was used for statistical analysis. When more than 2 treatments were analyzed a one-way ANOVA with multiple comparisons test was used, with correction for multiple comparisons with Tukey's test.

### Example 1: Switching the IgG1 constant domain to IgG3 to alter the avidity for Spike protein

Previously we generated 96 antibodies against the Wuhan Spike protein of SARS-CoV-2 through the isolation of Spike-reactive B-cells followed by single-cell sequencing ⁵. Of these 96 clones, 11 were strongly reactive to Spike protein, and all 11 were opsonic *in vitro.* These mAbs were produced in the IgG1 subclass irrespective of the original subclass of the patient B cells. Using PCR and homologous recombination, we exchanged the subclass-specific domains of the heavy chain in the heavy chain expression plasmid. We successfully generated 8 new heavy chain plasmids containing the original variable heavy domain and the IgG3 constant domain gene. There exist an extensive amount of allotypes for human IgG3 constant domain ⁹. The allotype used in this study was IGHG3^{∗}11 which, amongst other differences, contains a 62 amino acid long hinge ⁹. The heavy chain plasmids were transfected alongside the original light chain plasmid into Expi293F cells to produce the IgG3 variants of these antibodies (Fig. 1A).

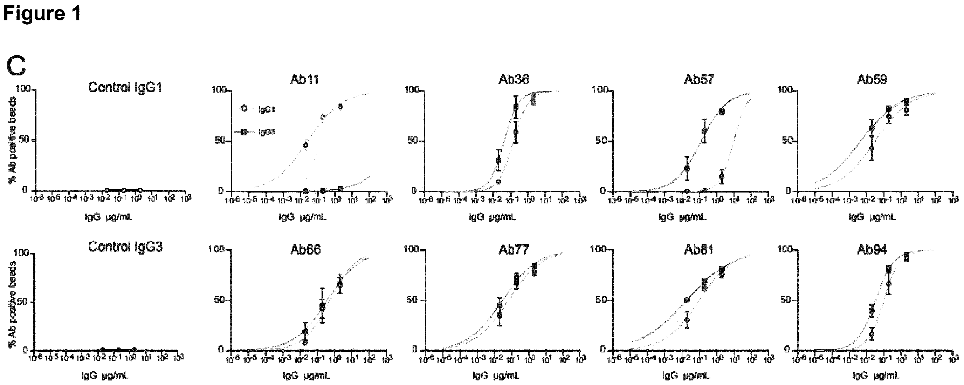

To measure the binding avidity of our newly produced mAbs, we conjugated biotinylated Spike protein to fluorescent streptavidin microsphere beads (1 µm) as a model for SARS-CoV-2 virions ⁵. We incubated our mAbs with the Spike beads before adding secondary fluorescent anti-Fab antibodies to detect IgG binding on the beads using flow cytometry as done previously ⁵ (Fig. 1B). The avidity is calculated by fitting a non-linear regression model to the dose-binding data. We observed that of our 8 IgG3 antibodies, 6 had an equivalent avidity to their IgG1 counterparts (Fig. 1C-D). However, there was a notable change for Ab57 where the IgG3 version exhibited an increase in avidity compared to the IgG1 parent (Fig. 1C-D). Ab11, on the other hand, showed severely reduced binding to Spike as an IgG3 antibody, while the IgG1 version retained high reactivity. It is important to point out the different clonal origins of Ab57 and Ab11. Ab57 and Ab11 had evolved as an IgG1 and IgG3 *in vivo.* Unfortunately, the information acquired from the sequencing of clone 11's IgG3 constant domain was incomplete. Thus, the allotype which existed in vivo in the donor patient can therefore not be determined. In both these cases, the original subclass (in the donor B cell), IgG3 for clone 11 and IgG1 for clone 57, bound with lower avidity to the spike protein than the subclass-switched mAb variants, IgG1, and IgG3 respectively (Fig. 1D).

To complement the flow cytometry-based binding data, we performed surface plasmon resonance (SPR) to study spike-mAb affinity in the context of the respective monovalent domains. For clones 11-81, we consequently used the receptor-binding domain (RBD) of the spike protein as an analyte, while we used the N-terminal domain (NTD) in the case of clone 94. These clones are from previous work known to bind to these respective domains ⁵. Neither Ab11, 36, nor 57 could bind to the RBD in this experimental setup for either subclass. For Ab59, Ab66, Ab77, Ab81, and Ab94, we observed no large differences in affinity (in terms of k_{A} and K_{D}) (Supp Fig. 2A), confirming the previous avidity data with spike bead flow cytometry. To measure the binding of Ab11, 36, and 57, we performed a similar experiment but replaced RBD as a ligand with the intact spike protein expressed as the trimer. Consistently with our previous findings, Ab11 was less reactive to the spike protein when expressed as IgG3, more than 25-fold less (Fig. 1E-F). However, we could observe no difference between IgG1 and IgG3 for clone 36. Clone 57 did show a 2-fold difference in apparent K_{D} in this experimental set-up (Fig. 1E-F). The results with SPR validated our data that the lab-generated form of Ab11 (as IgG1) and Ab57 (as IgG3) have a higher avidity for spike protein than their respective original native subclasses.

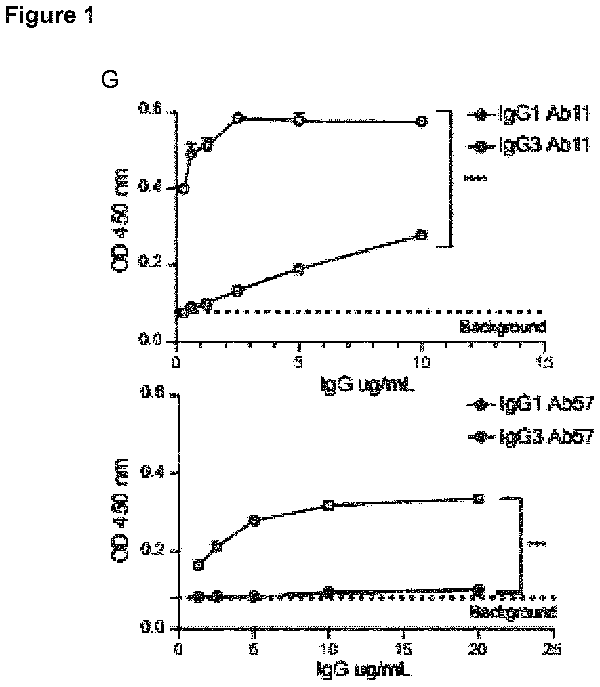

We further validated our Ab11 and Ab57 findings by coating ELISA plates with spike and testing mAb reactivity. The results from the ELISA experiments showed that Ab57 bound much stronger to spike when expressed as IgG3 with several-fold stronger signal over IgG1 (Fig 1G, Supp Fig. 3A). Also, unsurprisingly, that Ab11 IgG3 has a reduced reactivity to spike (Fig. 1G, Supp Fig. 3A) while IgG1 is strongly reactive. Taken together, the three different methods show that for clones 36, 59, 66, 77, 81, and 94, exchanging constant domain from IgG1 to IgG3 does not affect avidity to the spike-antigen. However, for clones 11 and 57 there is a large change in avidity when altering the constant domain. In these two instances, the best binding subclass was not the original subclass sourced from the donor B cell, as the artificially created subclass had a superior binding avidity (Fig. 1C-D, Fig. 1E-F and Fig. 1G).

### Example 2: IgG3 is a more potent inducer of Fc-mediated phagocytosis than IgG1

In our previous work, we utilized the THP-1 cell line to study how the IgG1 monoclonals promote Fc -mediated phagocytosis of spike beads ⁵. These findings correlated with protection in an animal model. Using a similar approach, we incubated opsonized spike-coated microsphere beads with THP-1 cells (Fig. 2A) to measure Fc-mediated phagocytosis. As our mAbs were all discovered by baiting B-cells with the original Wuhan-1 spike, we opted to use that antigen for our subsequent analysis. We stained the beads with a pH-sensitive dye (pHrodo green) to distinguish cell populations with internalized beads versus those that are only associated with beads (Fig. 2A).

On average, the IgG3 mAbs increased the percentage of cells with internalized beads more than 2-fold compared to their IgG1 counterparts. This was a consistent trend across all mAb clones (Fig. 2B), except for Ab11 IgG3, reflecting its reduced avidity. The largest fold-change increase when switching from IgG1 to IgG3 was seen for Ab36 IgG3 (3-fold) (Fig. 2B). In order of potency measured as % internalization, Ab94 (anti-NTD) IgG3 (47 %) was the most potent, followed by Ab59 (38 %) and Ab81 IgG3 (35 %) (Fig. 2B, Supp. Fig 4A). To put these values into context, the most potent IgG1 antibody, Ab94 IgG1, was similar to IgG3 Ab66 in potency (17 % vs 20 %) which was the second weakest IgG3 antibody. Beyond leading to a greater proportion of phagocytes with internalized beads, IgG3 also led to an increased amount of opsonized beads being associated with phagocytes compared to IgG1 (Fig. 2C, Supp. Fig 4A). Ab94 IgG3 led to a 7-fold increase over Ab94 IgG1. Ab81 IgG3 led to a 6-fold increase over its IgG1 counterpart while for Ab11 and Ab57 IgG3 no difference was observed. The difference between the IgG3 clones was much larger in this metric than percent of phagocytes with internalized beads. Ab94 IgG3 led to much more beads being associated with the THP-1 cells (73000 MFI) compared to the other antibodies, followed by Ab81 IgG3 (44000). Here again, the most potent IgG1 antibody, Ab59 (10500), was comparable to the weakest IgG3s.

Our results show that the proportion of cells with internalized beads and the amount of beads associated with cells do not fully correlate, and each clone exhibits a unique pattern in these metrics. Ab59 IgG3 and Ab81 IgG3 were equivalent in terms of how many percent of the cells internalized beads (38% vs 35%), but Ab81 IgG3 induced the uptake of far more beads, leading to a higher bead signal (44000 vs. 28000). Similarly, while Ab36 IgG3 was similar to Ab77 IgG3 in terms of the percent of internalizing cells (31% vs 30%), there was a large difference in bead-signal (29000 vs 17000). Moreover, Ab94 and Ab81 IgG3 stand out as the most potent mAbs in both metrics (Fig. 2B-C). Overall, exchanging constant domains to IgG3 enhances Fc-mediated phagocytosis for most of the tested mAb clones. Finally, it is worth noting that in the cases of clones 36, 57, 66, 77, and 81 that were IgG1s in their sourced form, the switched IgG3 forms were functionally superior to those originally encoded by the donor B cells.

### Example 3: IgG3 oligoclonal cocktails potentiate Fc-mediated phagocytosis compared to single monoclonals

Although the monoclonal IgG3s show potent opsonic ability as single mAbs, a mAb-based experimental setup does not represent an *in vivo* setting. In serum, multiple antibody clones contribute to overall phagocytic performance. To emulate this, we created cocktails of all 8 mAbs called OctomAb. To observe the contribution of the most potent clones (Ab94 and Ab81 IgG3), we created a cocktail consisting of these two mAbs named DuomAb. The remaining Ab11-Ab77 were put together in a third cocktail called HexamAb. To observe the effect of the cocktails, we lowered the multiplicity of prey (ratio of beads to phagocyte) from 30 to 15 and halved the concentration of mAbs (from 10 to 5 µg/ml) to not saturate the THP-1 cells with an abundance of prey and mAbs.

The IgG3 cocktails clearly enhanced the phagocytic potency of the IgG3 mAbs. There was a 3-fold enhancement of the proportion of cells with internalized beads for all three cocktails compared to their respective IgG1 cocktails (Fig. 2D). HexamAb, which does not contain the potent Ab94 and Ab81 IgG3, achieved similar phagocytosis as with monoclonal Ab94 IgG3 (32 % vs. 27 %). The observed differences were more pronounced (40000 vs 29000, respectively) when looking at bead signals (Fig. 2E). The IgG3 cocktails were much more potent in terms of the amount of associated beads per cell compared to their IgG1 counterparts. IgG3 DuomAb was 8-fold more potent, HexamAb 9-fold, and OctomAb performed 12-fold better than IgG1 (Fig. 2E). Most importantly, all three cocktails exceeded the most potent IgG3 mAb (Ab94) with a 38 % increase for HexamAb, 58% for OctomAb and 79% increase for DuomAb (Fig. 2E). Taken together the results show that oligoclonal antibody cocktails, even with lesser-performing mAbs, promote potent phagocytosis exceeding the best-performing mAb at the same concentration.

### Example 4: IgG3 monoclonals activate complement with high potency not exhibited by their IgG1 counterparts

We wanted to measure how well our IgG3 monoclonals and cocktails activate the classical complement pathway. We measured complement activation by adding 1% serum to opsonized spike beads. Using a secondary anti-C1q antibody (FITC) we could measure C1q deposition on the spike beads by flow cytometry. As clones 81 and 94 performed best in the previous assays, they were used as representatives for monoclonals. DuomAb and OctomAb cocktails were used to evaluate the benefit of cocktail use since these were the most potent of the cocktails. To avoid donor variability in terms of the presence of spike-reactive antibodies, we used pooled human serum depleted of antibodies as the source of complement.

The only mAb that by itself was able to induce C1q deposition was Ab94 IgG3 (Fig. 3A-B). When having both Ab81 IgG3 and Ab94 IgG3 together as DuomAb, they deposited C1q on the spike beads 2.5 times more than Ab94 by itself (38% vs 15%, Fig. 3A-B). This potent activation of complement was not seen with IgG1 DuomAb, indicating that it is a subclass-dependent phenomenon. The OctomAb containing all antibodies triggered C1q deposition to a similar degree as DuomAb, but only its IgG3 form. We then performed C1q deposition analysis as a function of mAb concentration. We calculated the EC₅₀ of DuomAb IgG3 and compared it to Ab94 IgG3. The resulting analysis shows that the addition of Ab81 IgG3 to Ab94 IgG3 increases the percentage of beads with C1q deposition more than 3-fold (EC₅₀ decreased from 37 to 11 µg/m!, Fig. 3C). When looking instead at the signal for C1q deposition, there was 20-fold increased efficacy (EC₅₀ decreased from 230 to 15 µg/ml) (Fig. 3D). This meant that the beads on which C1q had been deposited, having DuomAb led to a much stronger deposition compared to Ab94 only. Taken together, there is a clear increase in potency for anti-spike IgG3 mAbs compared to IgG1 in activating the classical complement pathway, a finding that is enhanced when the IgG3 mAbs are used as cocktails.

### Example 5: Neutrophil phagocytosis of spike-coated beads is dominated by Fc-mediated phagocytosis that is enhanced by IgG3 monoclonals and their cocktails

We decided to validate our phagocytosis findings with primary cells. We first used monocytes and determined how changing the multiplicity of prey (MOP) would affect internalization, as this is an essential experimental factor to consider in phagocytosis¹⁸. At low MOP (<1), monocytes barely internalize the spike beads, even with the potent OctomAb IgG3. However, at higher MOPs (1-10) the opsonic efficiency of mAbs is increased. To study the effects of IgG1 and IgG3 mAbs on primary cells, we therefore used a MOP of 5. We opted to use neutrophils instead of monocytes in subsequent experiments since they are more efficient at mediating phagocytosis and the most abundant phagocyte found in the blood ¹⁹.

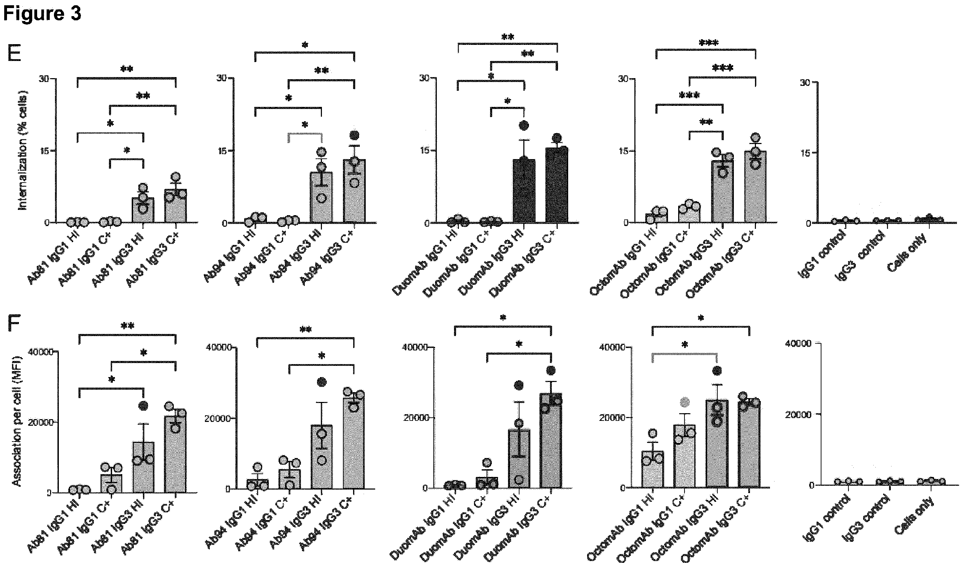

The results show only a minor difference between heat-inactivated (HI) serum and complement-active (C+) treatments. This indicates that Fc-mediated phagocytosis is the dominating mode of function for anti-spike monoclonals of both subclasses and that complement-mediated phagocytosis plays a minor role (Fig. 3E). When comparing subclasses, all IgG3 treatments were superior to their IgG1 counterparts in the proportion of neutrophils with internalized beads (Fig. 3E). The IgG3 cocktails were, as seen previously, equal to or slightly better than Ab94 IgG3 by itself (C+: OctomAb: 15.0± 1.6 %; DuomAb: 15.5± 1.1 % and Ab94: 13.1± 2.9 %) (Fig. 3E). OctomAb IgG1 (C+, 3.4± 0.3 %) was slightly better than both Ab81 (C+,0.4± 0.1 %) and Ab94 IgG1 (C+, 0.6± 0.1 %) which was not seen with THP-1 cells previously (Fig. 2D-E). However, the clear difference between OctomAb IgG1 and OctomAb IgG3 was seen again (3.4% vs 15.0%).

We also studied the quantity of beads being phagocytosed by the cells. Due to the lower MOP, more cells were unreactive compared to the THP-1 cells (MOP 30 vs 5). We analyzed the bead signal for the bead-positive neutrophils (APC+). This analysis reveals that all IgG3 treatments remained superior to their IgG1 counterparts (Fig. 3F), as seen with THP-1 cells. Interestingly, IgG1 OctomAb showed enhanced phagocytosis compared to the single IgG1 monoclonals, which was not seen with THP-1 cells (Fig. 2B, Fig. 3F). This shows that even IgG1 mAbs can benefit when used as cocktails to increase phagocytosis efficiency. Taken together the results show that IgG3 monoclonals and their cocktails outperform their IgG1 counterparts in promoting phagocytosis (Fc- and complementreceptor-mediated). Both subclasses benefit from using a cocktail-based oligoclonal approach for enhancing phagocytic performance.

To study the temporal dynamics of the phagocytic process elicited by the mAbs we utilized live fluorescence microscopy. We focused on comparing the best monoclonal Ab94 IgG3 and the oligoclonal OctomAb in both its IgG1 and IgG3 form. We did not heat-inactivate serum to be able to include CR-mediated phagocytosis. To be able to compare the flow cytometry data, we used neutrophils from the same donors (Fig. 3E-F) and the same experimental conditions (MOP 5). We looked at neutrophils with internalized beads and quantified the percentage of these cells, and the amount of beads they had internalized (Fig. 4A). OctomAb IgG3 stands out among treatments by yielding more cells with internalized beads during the course of the 60-minute experiment. We analyzed the efficiency of the IgG mAbs by calculating how long it would take for 50% of all the neutrophils to have internalized beads (ET₅₀). This analysis shows that OctomAb IgG3 was twice as fast at promoting internalization of spike beads compared to Ab94 IgG3 and OctomAb IgG1 (35 min, 66, and 62 min, respectively) (Fig. 4D). All mAb treatments are more efficient than the untreated negative control (165 min, Fig. 4D). Still, only OctomAb IgG3 led to a statistically significant difference. Interestingly, OctomAb IgG1 and Ab94 IgG3 performed equally over time (Fig. 4D). This reaffirms the results in Figure 3E-F, where an IgG1 cocktail enhanced more weakly performing IgG1 mAbs. While we previously used the median bead signal to quantify how many beads are being phagocytosed, live imaging allowed us to do this with greater precision (Fig. 4A). The result from this analysis shows that cells with internalized beads have, on average, more beads internalized in each neutrophil when the opsonin is IgG3 OctomAb and IgG3 Ab94, compared to IgG1 OctomAb (Fig. 4B-C). However, considering that IgG3 OctomAb activates more cells than IgG3 Ab94 (Fig.4D) (72% vs. 47% after 60 minutes), it promotes stronger bead-cell interaction overall. Together, these results highlight the benefit of using an oligoclonal cocktail of mAbs to increase the efficacy of mAbs for both IgG1 and IgG3.

### Example 6: Opsonizing anti-spike mAbs mediating Fc-mediated function protects against Sars-CoV-2 infection

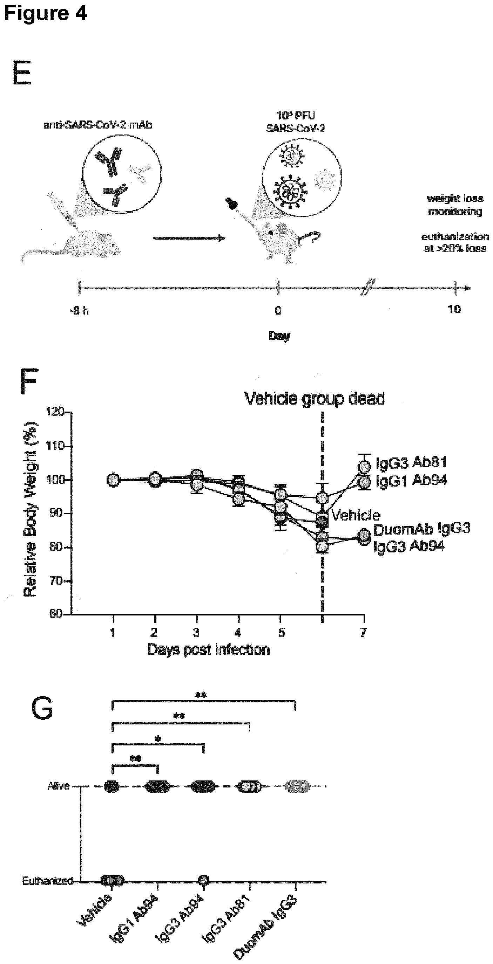

We complemented our THP-1 monocyte *in vitro* experiments with data from isolated primary human neutrophils from several donors. These experiments were done with microsphere beads ex *vivo.* We next wanted to validate the biological and potential clinical relevance of these Fc-mediated functions in protection against an authentic SARS-CoV-2 infection. The protective function of our mAbs was tested in an infection model with hACE2-K18 mice (Fig. 4E). We prophylactically injected each mouse intraperitoneally in one single dose (200 ug/mouse) with our mAbs (Ab94 IgG1, Ab94 IgG3, Ab81 IgG3, DuomAb IgG3 or vehicle control). Eight hours after mAb administration, we inoculated the mice intranasally with 1× 10⁵ PFU (SARS-CoV-2; Wuhan strain from Swedish isolate) of the virus. It is important to note that none of these clones have neutralizing activity, as verified by three independent methods⁵, and are considered strictly opsonizing.

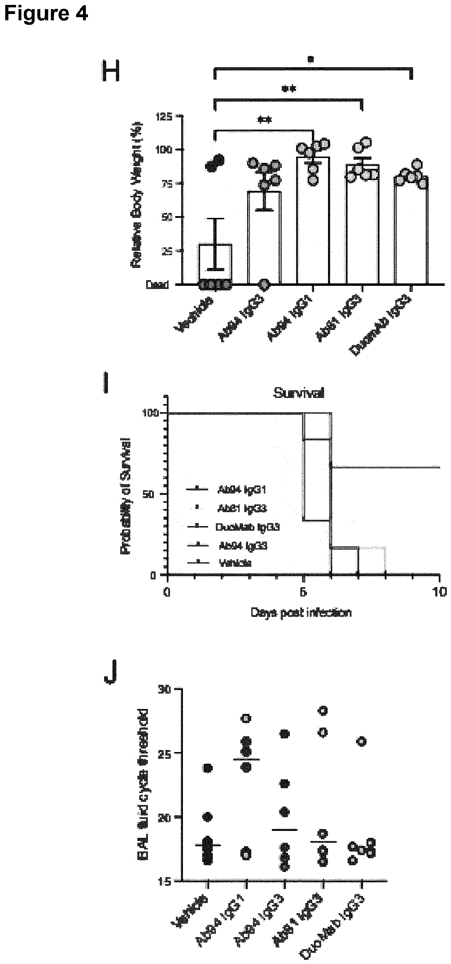

We theorized that all our antibodies would be protective compared to the vehicle control through their Fc-mediated effector functions. Measured each day over the course of the experiment, Ab94 IgG1 and Ab81 IgG3 showed lower weight loss compared to the vehicle control from days 0-7. More importantly, all the mAbs prevented death due to euthanization (which occurred when mice were experiencing inhumane clinical well-being or >20% body weight loss) (Fig. 4E). At day 7, all mice in the control group were dead, as opposed to the mAbs treated groups (highlighted in Figure 4F as survival *beyond* day 6 as a red-dashed line). This data is also reflected in the state of the mice in terms of survival status and body weight *at* day 6 (Fig. 4G). On day 6, four out of six mice in the control group were deceased, and one mouse in the Ab94 IgG3 group had been euthanized (Fig. 4G). No mice were deceased in the other antibody-treated groups at this time and maintained a healthy mean weight (Fig 4H). It is worth noting that only the Ab94 IgG1 and Ab81 IgG3 groups had survivors until day 10 (Fig. 4I). After euthanization, irrespective of which day the mice were deceased, bronchoalveolar lavage fluid (BAL) was harvested from all the mice in each control-group and analyzed for viral titers by qPCR measured as cycle threshold (CT). A higher value signifies a lower viral load. This analysis showed that the animals treated with all the mAbs, except DuomAb, had higher CT threshold values and, therefore, lower viral load upon euthanization (Fig. 4J). Ab94 IgG1 had the highest CT value (24.5), followed by Ab94 IgG3 (19) and then Ab81 IgG3 (18.1) compared to the control (17.8) (Fig 4I). Our data shows that mAb-treated mice had, in general, healthier mean weight, increased survival, and lower viral load in BAL-fluid. Taken together, our results show that opsonizing anti-spike antibodies, irrespective of the subclass, can be protective in a pre-clinical animal model of authentic SARS-CoV-2 infection. Except for Ab94 IgG1, which seemed to perform best in this experiment, it is difficult to draw conclusions on differences between the antibody treatments. Our data serves as a proof of concept that highlights the biological relevance of studying Fc-mediated effector functions in the context of SARS-CoV-2 adaptive immunity.

### Example 7: Discussion

This study focuses on how Fc-mediated immune functions of antibodies against spike protein can be potentiated. We find that altering the subclass leads to an increase in immune potency. Our data shows that IgG3 versions of an array of spike-specific antibodies are superior in function to their IgG1 counterparts - even if they were originally derived from an IgG1 B cell. Moreover, our data shows that Fc-mediated function is greatly enhanced when antibodies are combined into cocktails.

An incredibly large number of IgG antibody clones circulates in serum at any given time. Serum is thus a versatile polyclonal mix of antibodies which after infection, becomes geared to antigenspecific antibodies. In our current work, we show that in the hierarchy of efficacy, single IgG3 antibodies (e.g. Ab94) can be very efficient at mediating Fc-mediated immune function. Multiple lesser efficacious antibodies, however, when pooled together (Hexamab) can be equally powerful in mediating immune function. This could be due to enhanced Fc-receptor activation due to clustering of multiple Fcs on the antigen trimer, theorized to be important for efficient phagocytosis ²⁵⁻²⁷. The findings regarding the importance of cocktails also translated into an increased efficacy in activating complement deposition.

C1q requires multiple Fcs formed into a hexamer to be activated ²⁸. Our results suggest that IgG3 cocktails are more efficient at forming hexamer-clusters than IgG1 cocktails. Another important thing to note is that the cocktail benefit was more pronounced when used to opsonize spike beads for neutrophils compared to THP-1 cells. This could be attributed to neutrophils being professional phagocytes with a wider repertoire of phagocytic receptors ²⁹. We have shown how we can potentiate immune function by using multiple opsonic mAbs in combination. We believe that considering an IgG3 platform for future mAb therapy against emerging variants of concerns and other pathogens could be a promising approach, as IgG3s are more functionally active *in vitro* than IgG1, especially in cocktail form.

### TIL BACKGROUND SECTION :

There exist some biological limitations to IgG3's utility as a drug *in vivo.* Of the four subclasses, IgG3 has the shortest half-life ⁹ due to lower affinity to the neonatal Fc-receptor ⁹. Through Fc-engineering, the affinity to the Fc-neonatal receptor can be increased without diminishment to Fc-effector functions ^{30,31}. This alteration has been shown to be crucial for *in vivo* protection in a mouse model ³⁰. Oligoclonal IgG3 mAbs should therefore be modified to express more beneficial pharmacokinetics for optimal therapeutic effect in preclinical testing.

This issue of lower biological activity *in vivo* for IgG3 was possibly reflected in clone 94. We believe that the performance of IgG3 Ab94 compared to its IgG1 counterpart could possibly be explained due to shorter half-life or potentially less effective Fc interactions. Half-life of human IgG is shorter in mice compared to humans - on the order of days rather than weeks - and after 8 h, when the virus was administered, a larger proportion of the IgG3 subclasses are likely eliminated compared to IgG1, and this can also differ depending on the mAb³². It is worth noting that Ab81 IgG3 was close to Ab94 IgG1 in protective effect. We attribute this to it having a higher affinity than both subclasses of clone 94, thus possibly being more functionally active *in vivo.* We believe the hACE2-K18 model is a good model for showing the biological relevance of Fc-effector functions against SARS-CoV-2 infections as a proof of concept. However, the biological transferability of studying human subclasses in this model and predicting the outcome in humans is much more limited and warrants caution. Amongst other notable differences, mice have different Fc-receptors expressed on their phagocytes compared to human phagocytes ³³. These Fc-receptors, although closely resembling the affinities seen in human Fc-receptors ³⁴, are understudied, especially in the context of IgG3 and its many allotypes. Those studies that have investigated human subclass affinities to mouse Fc-gamma receptors have observed that human IgG3 binds as efficiently as IgG1. However, despite this, human IgG3 has been shown to have reduced *in vitro* function by mouse phagocytes compared to human IgG1^{35,36}. This is contrary to *in vitro* studies with human phagocytes, where human IgG3 has been shown to have more potent function compared to IgG1^{12-14, 30}. We believe that more work is needed to better link *in vitro* assays, in *vivo* animal models, and potential clinical benefits when studying human IgG subclasses as therapeutics. Progress has been made in generating mice with humanized immune systems to address these discrepancies across species ^{37,38} but the models currently available are not without their limitations either ³⁹, including model availability, relative complexity, and effects of human donor variation required for engrafting human tissue or cells. Nonetheless, from the perspective of understanding the role of Fc-mediated function in SARS-CoV-2 immunity, our *in vivo* data support that Fc-effector functions of non-neutralizing mAbs can be strongly protective against Sars-CoV-2 infection. In this regard generating human IgG3s is a promising alternative to IgG1, especially if its pharmacokinetic traits with shorter half-life are addressed with suitable engineering.

Additionally, we present evidence that exchanging constant domains impacts the ability of the variable domain to bind to the spike protein. Ab11 IgG3 exhibits reduced avidity compared to Ab11 IgG1. Ab57 IgG3, on the other hand, binds better than IgG1. This is interesting because clone 57 is originally IgG1, and clone 11 is originally IgG3 (according to sequencing data) ⁵. An IgG1 antibody is formed as the IgG3 constant domain gene is deleted during the class-switch recombination process ^{9, 40}. Thus, IgG1 can evolve from an IgG3 but an IgG3 cannot develop from a clone producing an IgG1 antibody. Although an IgM can be switched directly to an IgG1 antibody without having an lgG3 intermediate, a B-cell that makes an IgG3 has not evolved to produce an IgG1. Following this, clone 11 was sourced from an IgG3 producing B-cell, meaning Ab11 IgG1 has not been subclass-switched in that B-cell before. We find it interesting then that the artificial Ab11 IgG1 has much higher avidity to spike than the original IgG3. In addition, Ab57 IgG3 shows that a theoretically less evolved mAb is more reactive to the antigen than the naturally evolved Ab57 IgG1. These examples raise the question if the most reactive mAb being expressed by a B cell is the one circulating in B cells at any given point at the 6-8 week window post infection (in Bahnan et al 2022, patient B cells were acquired 6-8 weeks after initial SARS-CoV-2 infections ⁵). Our results show that the change of avidity does not always follow the anticipated subclass switch hierarchy of the IGHV locus. However, our data is based only on eight clones, but raises new questions which are worth considering. Our results further add to growing evidence ^{10, 41 42} that antibody constant and variable domains influence each other. We further showed that the Fc-effector function was, irrespective of the original subclass, most potent in the IgG3 subclass. Future work with subclass-switched mAbs is therefore warranted to broaden our understanding of variable and constant domain interactions and how that influences immune function in particular disease.

Overall, this study highlights the benefit of subclass-switching mAbs to IgG3 and using them in oligoclonal cocktails for enhancing *in vitro* opsonic function. These results are important for the future development of therapeutic mAbs when Fc- and CR-mediated phagocytosis is desirable. In the context of SARS-CoV-2 and its VOCs, using opsonic monoclonal therapeutics could constitute a promising avenue for further research, especially since they are less dependent on binding to mutation-prone epitopes in the RBD. Indeed our study shows the promise of using non-neutralizing but opsonic mAbs against SARS-CoV-2 infections and using IgG3 mAbs instead of IgG1s due to their potent Fc-effector functions. The differences between our *in vitro* data with human phagocytes and the outcome in animals stresses the importance of future work on optimizing preclinical models for therapeutic development of mAbs. Additionally, the study presented here is a reminder of the intricacies involved in B cell subclass switching and highlights the importance of different subclasses in an immune response.

### List of reference.

1. Hu, B., Guo, H., Zhou, P. & Shi, Z. L. Characteristics of SARS-CoV-2 and COVID-19. Nat. Rev. Microbiol. 19, 141-154 (2021).
2. Chen, P. et al. SARS-CoV-2 Neutralizing Antibody LY-CoV555 in Outpatients with Covid-19. N. Engl. J. Med. 384, 229-237 (2021).
3. Tortorici, M. A. et al. Ultrapotent human antibodies protect against SARS-CoV-2 challenge via multiple mechanisms. Science (80-. ). 370, 950-957 (2020).
4. Yamayoshi, S. et al. Antibody titers against SARS-CoV-2 decline, but do not disappear for several months. EClinicalMedicine 32, 100734 (2021).
5. Bahnan, W. et al. Spike-Dependent Opsonization Indicates Both Dose-Dependent Inhibition of Phagocytosis and That Non-Neutralizing Antibodies Can Confer Protection to SARS-CoV-2. Front. Immunol. 12, 1-17 (2022).
6. Winkler, E. S. et al. Human neutralizing antibodies against SARS-CoV-2 require intact Fc effector functions for optimal therapeutic protection. Cell 184, 1804-1820.e16 (2021).
7. Ullah, I. et al. Live imaging of SARS-CoV-2 infection in mice reveals that neutralizing antibodies require Fc function for optimal efficacy. Immunity 54, 2143-2158.e15 (2021).
8. Kaplonek, P. et al. mRNA-1273 vaccine-induced antibodies maintain Fc effector functions across SARS-CoV-2 variants of concern. Immunity 55, 355-365.e4 (2022).
9. Vidarsson, G., Dekkers, G. & Rispens, T. IgG subclasses and allotypes: From structure to effector functions. Front. Immunol. 5, 1-17 (2014).
10. Torres, M. & Casadevall, A. The immunoglobulin constant region contributes to affinity and specificity. Trends Immunol. 29, 91-97 (2008).
11. Bruhns, P. et al. Specificity and affinity of human Fcy receptors and their polymorphic variants for human IgG subclasses. Blood 113, 3716-3725 (2009).
12. Bindon, B. Y. C. I., Hale, G. & Bruggemann, M. Human monoclonal igg isotypes differ in complement activating function at the level of c4 as well as C1q Monoclonal antibodies have attracted much interest as agents to kill unwanted cells for therapeutic purposes . A limitation to the scope of mAb therapy. 168, (1988).
13. Giuntini, S., Reason, D. C. & Granoff, D. M. Combined roles of human IgG subclass, alternative complement pathway activation, and epitope density in the bactericidal activity of antibodies to meningococcal factor H binding protein. Infect. Immun. 80, 187-194 (2012).
14. Chu, T. H. et al. Hinge length contributes to the phagocytic activity of HIV-specific IgG1 and IgG3 antibodies. PLoS Pathog. 16, 1-25 (2020).
15. Foss, S. et al. Potent TRIM21 and complement-dependent intracellular antiviral immunity requires the IgG3 hinge. Sci. Immunol. 7, eabj1640 (2022).
16. Kallolimath, S. et al. Highly active engineered IgG3 antibodies against SARS-CoV-2. Proc. Natl. Acad. Sci. U. 5. A. 118, 3-4 (2021).
17. Chen, Y. et al. Engineered ACE2-Fc counters murine lethal SARS-CoV-2 infection through direct neutralization and Fc-effector activities. Science Advances, 8, 28 (2022).
18. de Neergaard, T., Sundwall, M., Wrighton, S. & Nordenfelt, P. High-Sensitivity Assessment of Phagocytosis by Persistent Association-Based Normalization. J. Immunol. 206, 214-224 (2021).
19. Rabinovitch, M. Professional and non-professional phagocytes: an introduction. Trends Cell Biol. 5, 85-87 (1995).
20. VanBlargan, L. A. et al. An infectious SARS-CoV-2 B.1.1.529 Omicron virus escapes neutralization by therapeutic monoclonal antibodies. Not. Med. 28, 490-495 (2022).
21. Harvey, W. T. et al. SARS-CoV-2 variants, spike mutations and immune escape. Nat. Rev. Microbiol. 19, 409-424 (2021).
22. Beaudoin-Bussières, G. et al. A Fc-enhanced NTD-binding non-neutralizing antibody delays virus spread and synergizes with a nAb to protect mice from lethal SARS-CoV-2 infection. Cell Rep. 38, (2022).
23. Liu, H. et al. A combination of cross-neutralizing antibodies synergizes to prevent SARS-CoV-2 and SARS-CoV pseudovirus infection. Cell Host Microbe 29, 806-818.e6 (2021).
24. Dussupt, V. et al. Low-dose in vivo protection and neutralization across SARS-CoV-2 variants by monoclonal antibody combinations. Nat. Immunol. 22, 1503-1514 (2021).
25. Radaev, S. & Sun, P. Recognition of immunoglobulins by Fcy receptors. Mol. Immunol. 38, 1073-1083 (2002).
26. Woof, J. M. & Burton, D. R. Human antibody-Fc receptor interactions illuminated by crystal structures. Nat. Rev. Immunol. 4, 89-99 (2004).
27. Yang, D., Kroe-Barrett, R., Singh, S., Roberts, C. J. & Laue, T. M. IgG cooperativity-Is there allostery? Implications for antibody functions and therapeutic antibody development. MAbs 9, 1231-1252 (2017).
28. Diebolder, C. A. et al. Complement is activated by IgG hexamers assembled at the cell surface. Science (80-. ). 343, 1260-1263 (2014).
29. Nordenfelt, Pontus; Tapper, H. Phagosome dynamics during phagocytosis by neutrophils. J. Leukoc. Biol. 90, 271-284 (2011).
30. Stapleton NM, Andersen JT, Stemerding AM, Bjarnarson SP, Verheul RC, Gerritsen J, et al. Competition for FcRn-mediated transport gives rise to short half-life of human IgG3 and offers therapeutic potential. Nature Communications. 2011;2(1).
31. Ko S, Park S, Sohn MH, Jo M, Ko BJ, Na JH, Yoo H, Jeong AL, Ha K, Woo JR, Lim C, Shin JH, Lee D, Choi SY, Jung ST. An Fc variant with two mutations confers prolonged serum half-life and enhanced effector functions on IgG antibodies. Exp Mol Med. 2022 Nov;54(11):1850-1861. doi: 10.1038/s12276-022-00870-5.
32. Bazin R, Boucher G, Monier G, Chevrier MC, Verrette S, Broly H, Lemieux R. Use of hu-IgG-SCID mice to evaluate the in vivo stability of human monoclonal IgG antibodies. J Immunol Methods. 1994 Jun 24;172(2):209-17. doi: 10.1016/0022-1759(94)90108-2.
33. Pierre Bruhns; Properties of mouse and human IgG receptors and their contribution to disease models. Blood 2012; 119 (24): 5640-5649.
34. Gillian Dekkers, Arthur E. H. Bentlage, Tamara C. Stegmann, Heather L. Howie, Suzanne Lissenberg-Thunnissen, James Zimring, Theo Rispens & Gestur Vidarsson (2017) Affinity of human IgG subclasses to mouse Fc gamma receptors, mAbs, 9:5, 767-773.
35. Steplewski Z, Sun LK, Shearman CW, Ghrayeb J, Daddona P, Koprowski H. Biological activity of human-mouse IgG1, IgG2, IgG3, and IgG4 chimeric monoclonal antibodies with antitumor specificity. Proc Natl Acad Sci U S A 1988; 85:4852-6.
36. Overdijk MB, Verploegen S, Ortiz Buijsse A, Vink T, Leusen JHW, Bleeker WK, Parren PWHI. Crosstalk between human IgG isotypes and murine effector cells. J Immunol 2012; 189:3430-8.
37. Smith P, DiLillo DJ, Bournazos S, Li F, Ravetch JV. Mouse model recapitulating human Fcy receptor structural and functional diversity. Proc Natl Acad Sci USA. 2012;109(16):6181-6186.
38. rady JM.,Phelps M., Macdonald SW., Lam EC et al. Antibody-mediated prevention of vaginal HIV transmission is dictated by IgG subclass in humanized mice. Science translational medicine 14 (665) (2022).
39. Allen, T.M., Brehm, M.A., Bridges, S. et al. Humanized immune system mouse models: progress, challenges and opportunities. Nat Immunol 20, 770-774 (2019).
40. Chu, T. H., Patz, E. F. & Ackerman, M. E. Coming together at the hinges: Therapeutic prospects of IgG3. MAbs 13, (2021).
41. Janda, A., Eryilmaz, E., Nakouzi, A., Cowburn, D. & Casadevall, A. Variable region identical immunoglobulins differing in isotype express different paratopes. J. Biol. Chem. 287, 35409-35417 (2012).
42. Janda, A., Bowen, A., Greenspan, N. S. & Casadevall, A. Ig constant region effects on variable region structure and function. Front. Microbiol. 7, 1-10 (2016).

## Claims

1. An engineered antibody comprising an IgG3 hinge and a variable domain having a viral spike protein binding part.

2. The engineered antibody according to claim 1, wherein the Fc region is an IgG3 region.

3. The engineered antibody according to claim 2, wherein the Fc region comprises the IgG3 heavy chain constant domain SEQ ID NO:1 or a variant thereof having at least 90% identity, at least 95% identity, at least 98% identity or 100% identity to SEQ ID NO:1.

4. The engineered antibody according to claim 2, wherein the IgG3 region comprises the three substitutions R435H, N392K and M397V.

5. The engineered antibody according to any of claims 3-4, wherein said IgG3 region comprises a polypeptide having at least 95% identity or at least 98% identity to the modified IgG3 heavy chain constant domain of SEQ ID NO:2.

6. The engineered antibody according to any of claims 3-5, wherein said IgG3 region comprises the modified IgG3 heavy chain constant domain of SEQ ID NO:2.

7. The engineered antibody according to claim 1, wherein the Fc region is an IgG1 region.

8. The engineered antibody according to claim 7, wherein said engineered antibody comprises an IgG1 constant domain engineered with IgG3 hinge, SEQ ID NO:3.

9. The engineered antibody according to any of claims 1-8, wherein said viral spike protein is from a corona virus.

10. The engineered antibody according to any of claims 1-9, wherein said viral spike protein is a SARS-Cov-2 spike protein.

11. The engineered antibody according to any of claims 1-10, wherein said IgG3 hinge comprises an amino acid sequence which has at least 90% identity, at least 95% identity, at least 98% identity or 100% identity to the amino acid sequence ELKTPLGDTTHTCPRCPEPSKCDTPPPCPRCPEPSKCDTPPPCPRCPEPSKCDTPPPCPRCP (SEQ ID NO:7).

12. The engineered antibody according to any of claims 1-10, wherein said IgG3 hinge comprises an amino acid sequence which is at least 90% identity, at least 95% identity, at least 98% identity or 100% identity to the amino acid sequence X-(Y)n, wherein
X is ELKTPLGDTTHTCPRCP (SEQ ID NO:8),
Y is EPSKCDTPPPCPRCP (SEQ ID NO:9), and
n is an integer in the range from 2 to 8, preferably in the range from 2 to 5, more preferably in the range from 2 to 4.

13. The engineered antibody according to any of claims 1-12, which promotes opsonisation.

14. The engineered antibody according to any of claims 1-12, which does not bind to neutralising epitopes.

15. The engineered antibody according to any of claims 1-14, wherein said engineered antibody is selected from the group consisting of Ab11, Ab36, Ab57, Ab59, Ab66 and Ab77.

16. A composition comprising at least two different engineered antibodies as defined in any of claims 1-15.

17. The composition according to claim 16, comprising from 3 to 15, from 3 to 12, from 3 to 9, from 5 to 15, from 5 to 10, or from 6 to 10 different engineered antibodies as defined in any of claims 1-15.

18. A nucleic acid molecule encoding a protein comprising the engineered antibody as defined in any of claims 1-15.

19. A cell comprising the nucleic acid as defined in claim 18.

20. Use of the engineered antibody as defined in any of claims 1-15, for preventing, treating or alleviating a viral infection caused by a virus comprising said spike protein.

21. Use of the engineered antibody as defined in any of claims 1-15, for Fc-mediated phagocytosis of a virus comprising said spike protein.

22. Use of the composition as defined in any of claims 16-17, for activating the complement pathway.

23. A method for preparing an engineered antibody as defined in any of claims 1-15, comprising the steps:
- immunization of a host organism with said viral spike protein or a protein comprising said viral spike protein,
- isolation of at least one viral spike protein reactive B-cell to isolate an IgG1 antibody binding to said viral spike protein,
- preparing at least one recombinant DNA molecule which encodes a modified version of said IgG1 antibody comprising the replacing of the IgG1 subclass specific domain of said isolated IgG1 antibody with the IgG3 subclass specific domain,
- expressing said at least one recombinant DNA molecule in a suitable host cell,
to obtain said engineered antibody.

24. A method for preparing an engineered antibody as defined in any of claims 1-15, comprising the steps:
- immunization of a host organism with said viral spike protein or a protein comprising said viral spike protein,
- isolation of at least one viral spike protein reactive B-cell to isolate an IgG1 antibody binding to said viral spike protein,
- preparing at least one recombinant DNA molecule which encodes a modified version of said IgG1 antibody, said modification comprising the replacing of the IgG1 hinge of said isolated IgG1 antibody with an IgG3 hinge,
- expressing said at least one recombinant DNA molecule in a suitable host cell,
to obtain said engineered antibody.
